# EUROPEAN PATENT APPLICATION

(11) **EP 2 110 367 A1**
(43) Date of publication of application: **21.10.2009**
(21) Application number: 08103530.5
(22) Date of filing: 14.04.2008
(51) Int. Cl.: C07B 59/00, C07B 63/02

(54) **Purification strategy for direct nucleophilic procedures**

(71) Applicant: Bayer Schering Pharma Aktiengesellschaft, 13353 Berlin (DE)
(72) Inventor: Berndt, Mathias, 12163, Berlin (DE); Cyr, John E., Fairfield, CA 94534 (US); Graham, Keith, 10115, Berlin (DE); Srinivasan, Ananth, 10629, Berlin (DE)

(57) **Abstract**

The present invention provides novel and advantageous processes for preparing and purifying chemical compounds such as pharmaceuticals. The processes comprise a nucleophilic substitution reaction with a moiety X wherein the leaving group L of a substrate S in the reaction is covalently attached to a purification moiety M. This concept offers a convenient and time-saving way to purify the desired product S-X from non-reacted precursors S-L-M and by-products L-M.

## Description

### Field of the Invention

The invention generally relates to the preparation of pharmaceuticals. In particular, this invention relates to processes and kits for carrying out an efficient "liquid phase" nucleophilic substitution reaction with a nucleophilic reagent X on a precursor targeting substrate S comprising a purification moiety M attached via a leaving group L to the targeting substrate. The methods and kits of the present invention allow a simple purification of the desired pharmaceutical S-X from non-reacted precursors and by-products still containing said purification moiety M.

### Background of the Invention

In the preparation of many pharmaceuticals, including radiohalogenated pharmaceuticals, nucleophilic substitution reactions as depicted in Scheme 1 are useful and commonly employed.

Scheme 1: **X + S-L → S-X + L**

wherein S is a targeting substrate,
X is a nucleophilic reagent and
L is a leaving group.

For example, US 5,565,185 discloses a non-carrier process of radiolabelling meta-iodobenzylguanidine (MIBG) by halodestannylation. However, the process is disadvantageous in that a number of impurities remain in solution with the radiolabelled MIBG. In particular, toxic tin by-products remain in solution and must be separated before the radiolabelled MIBG is ready for use.

Strategies to remove by-products, such as excess precursors had to be established for successful (radio-) synthesis and subsequent safe administration of compounds of clinical interest. Such reactions often employ non-radioactive organic precursors in amounts that are in large excess relative to the amount of the radiolabelling agent used. Excess precursors must then be removed from the reaction mixture before the radiolabelled compound can be applied to a patient for diagnostic and/or therapeutic applications.

In the case of radiohalogen pharmaceuticals, X can generally be easily separated from the other species in the reaction mixture using for example alumina solid phase extraction. Moreover, those of skill in the art are generally aware of methods to remove other radiolabelled, nucleophilic species such as ¹¹C-compounds or nucleophilic compounds in general, using standard purification protocols.

It is, however, generally more difficult to separate S-X and S-L. In many cases, it is particularly important to separate S-X from unlabelled targeting substrate S-L, because S-L can compete with and therefore interfere with binding of S-X to its target. If this competition occurs, this effect may lead to sub-optimal performance characteristics of the radiopharmaceutical. This is particularly the case for receptor-binding (i.e. specific targeting) radiopharmaceuticals.

The purification of S-X from S-L is commonly accomplished by employing a chromatographic, e.g., HPLC, purification procedure. However, this technique requires specialized equipment and can moreover be tedious and time-consuming. Considering the half-life of most clinically useful radioisotopes, it is desirable to complete the radiosynthesis and purification prior to administration to a patient as rapidly as possible. For example, the half-life of ¹⁸F is 110 minutes and ¹⁸F-labelled targeting substrates are therefore synthesized and purified within one hour of clinical use.

In view of the above, it is readily apparent that there is a need in the art for purification techniques which offer rapid and efficient separation of unwanted species from the final pharmaceutical S-X.

Since the introduction of the Merrifield method for peptide synthesis, insoluble polymer supports have been incorporated into numerous synthetic methodologies to facilitate product purification. In the solid phase peptide synthesis method, the nucleophile of a substitution reaction is covalently linked to a solid phase resin as shown in Scheme 2. Following the substitution reaction, the excess substrate S-L and the displaced leaving group L are easily separated from the resin-bound product X-S-Resin by filtration.

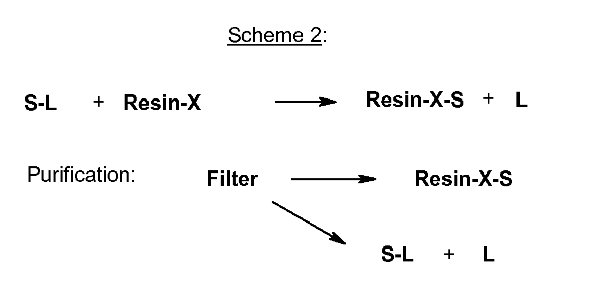

WO 2003/0012730 discloses an alternative radiohalogenation method in which the substrate of the substitution reaction is covalently linked to a solid phase resin through the leaving group as shown in Scheme 3.

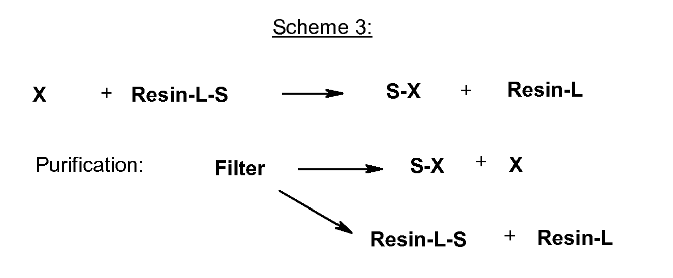

With this strategy, a radiolabelling agent X is reacted with the solid phase-supported substrate to form S-X, which is conveniently separated from non-reacted Resin-L-S and resin-bound leaving group Resin-L by washing and filtering off the resin.

Solid phase processes for the production of ¹⁸F-radiolabelled tracers suitable for use as positron emission tomography radiotracers are for example disclosed in WO 2003/002157.

Although solid phase-supported nucleophilic substitution technologies can simplify purification steps substantially, they suffer from the inherent drawback that heterogeneous reaction conditions are usually less efficient , leading to poor radiochemical yields and slower reaction times compared to reactions carried out in solution, i.e., without a solid support.

Alternative radiolabelling strategies that utilize homogeneous substitution reaction conditions are for example disclosed in WO 2005/107819 and in a scientific publication of Donavan et al (J. Am. Chem. Soc., 2006, 128, 3536-3537).

WO 2005/107819 relates to the purification of a radiolabelled tracer vector-X-R* resulting from a substitution reaction of R* for Y on the substrate vector-X-Y, using a solid support-bound scavenger group (scavenger resin). The scavenger resin Z-resin undergoes a similar substitution reaction on the non-reacted substrate vector-X-Y to displace Y and generate vector-X-Z-resin, which can be filtered off from the product vector-X-R* (which remains in solution). Hence, the purification procedure separates product from unreacted precursor. Also, the scavenger resins are only designed to displace the moiety Y of the reactive group. In other words, this approach is limited to remove non-reacted precursors but does not allow a simultaneous removal of **Y** leaving group from the product. Furthermore, the reactive moiety Z of the scavenger resin described in WO 2005/107819 is limited only to groups that are good substitution agents for **Y**.

Donavan et al. (*loc. cit*.) describe a "homogeneous" soluble supported procedure for electrophilic radioiodine substitution utilizing a fluorine-rich soluble support wherein a leaving group is linked to a perfluorinated moiety. The radioiodinated product was isolated from both unreacted substrate and the leaving group based on the strong affinity of the perfluorinated moiety for other perfluorinated species.

Although this homogeneous substitution procedure with fluorous-based purification has been demonstrated effective for radioiodination, it is not likely to be as useful for, e.g., ¹⁸F radiolabelling or nucleophilic reactions in general, because the Sn substrates are specific for electrophilic substitutions. Electrophilic ¹⁸F substitution is not often performed because the radiofluorine gas [¹⁸F] F₂ is not readily available, and because it has low specific activity (resulting from added [¹⁹F] F₂ carrier gas). Furthermore, fluorous-based purification using more preferred (higher specific activity) nucleophilic substitution reactions with [¹⁸F] fluoride are expected to be problematic in view of the exchange of ¹⁸F for cold (¹⁹F) fluorine from the perfluorinated moiety. Such fluorine exchange reactions are well-known, and can lead to lower radiochemical yields and poor specific activity of the radiopharmaceutical.

From the above, it is evident that other soluble-supported purification strategies for *inter alia* radiohalogen pharmaceuticals are needed which are easy to use and provide a broader applicability compared to the prior art described above. It would therefore be useful to develop alternative strategies for purifying, e.g., radiohalogen-containing pharmaceuticals which do not require HPLC purification and moreover reliably ensure efficient separation of S-X from unreacted precursor compounds S-L as well as from the leaving group by-product L.

### Summary of the Invention

The present invention generally relates to novel processes and kits for the preparation and purification of pharmaceuticals. In particular, this invention relates to methods and kits for carrying out an efficient liquid phase nucleophilic substitution reaction for preparing pharmaceuticals, including radiopharmaceuticals, and to a subsequent purification of the product using the unique properties of a purification moiety M which is attached to the leaving group of a precursor substrate. The purification process of the present invention separates the substitution product from non-reacted precursor molecules and from displaced leaving groups of a nucleophilic substitution reaction.

The processes and the products they produce are advantageous in several respects. The processes allow a simple and effective separation of non-reacted precursors and by-products from the desired main product using standard laboratory manipulations and without the need for sophisticated purification equipment. In addition, the separation procedures as described herein according to the method of the present invention are often much more convenient, flexible and most importantly less time consuming, which is a great advantage for example in the handling of clinically employed short-lived radiopharmaceuticals such as ¹⁸F-labelled pharmaceuticals.

Accordingly, the present invention relates in a first aspect to a process for preparing a pharmaceutical S-X, wherein the moiety L-M of a precursor species S-L-M is replaced by a reactant X through a liquid phase nucleophilic substitution to form said pharmaceutical S-X and a species L-M, wherein S is a targeting substrate; L is a leaving group covalently attached to M and further covalently attached to S prior to said nucleophilic substitution reaction; and M is a purification moiety that is covalently bound to L and has characteristics that allow species that contain said purification moiety M to be separated from other species that do not contain said purification moiety M. Optionally, S-X is further reacted to yield the final product S-X'.

In a second aspect, the invention relates to a process for preparing and purifying a pharmaceutical S-X, wherein the moiety L-M of a precursor species S-L-M is replaced by a reactant X through a liquid phase nucleophilic substitution to form said pharmaceutical S-X and a species L-M, optionally, wherein S-X is further reacted to yield the final product S-X'; and wherein any species that still contain said purification moiety M (M-containing species) are selectively separated from species not containing said purification moiety M, preferably S-X, by a purification procedure, e.g., as set out herein in further detail below.

In certain embodiments, the nucleophilic substitution reaction of S-L-M to S-X is a soluble supported reaction.

In a third aspect, the present invention relates to a process for purifying a pharmaceutical S-X from a liquid phase reaction mixture comprising S-X, S-L-M, and optionally L-M by selectively separating any species which contain said purification moiety M from said pharmaceutical S-X using a purification procedure. Suitable purification procedures according to the present invention will be described in further detail herein below.

In preferred embodiments, the liquid phase nucleophilic substitution reaction is a homogeneous nucleophilic substitution reaction, i.e. the reaction is carried out in a single liquid phase.

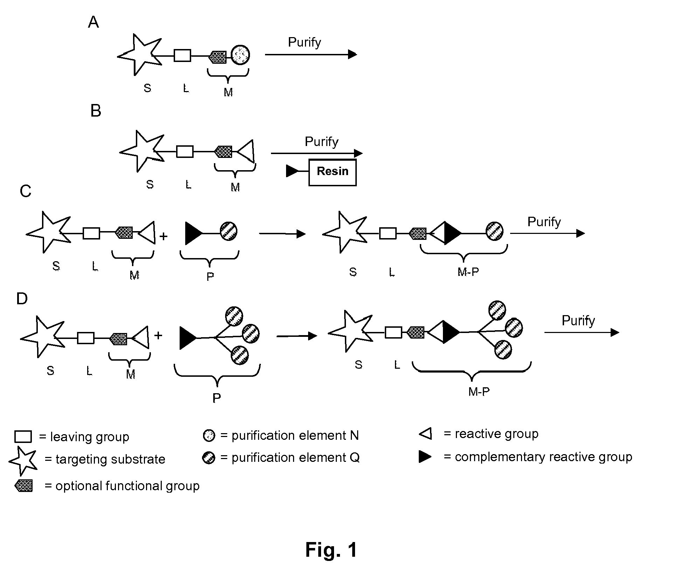

The purification processes of the invention are depicted schematically in Figure 1. In preferred embodiments, the purification, i.e., separation of M-containing species from non-M-containing species is accomplished directly on the M-containing species (cf. Figure 1A-1B), e.g. by liquid-liquid or solid-liquid phase extraction, by adjusting the reaction mixture to conditions so that M-containing species precipitate, by separation of M-containing species based on size exclusion, and/or by covalent binding of a reactive group on the purification moiety M to a complementary reactive group, e.g., attached to a resin (Figure 1 B). Alternatively, the purification procedure may also comprise a 2-step process wherein the complementary reactive group is covalently attached to a second purification moiety P resulting in a species M-P, and subsequent purification of the M-P product proceeds according to a purification method as mentioned above (cf. Figure 1C-1D).

Furthermore, another aspect of the present invention relates to kits for carrying out a nucleophilic substitution and/or purification according to the present invention. In one embodiment, a kit according to the invention comprises at least a purification moiety M or a moiety L-M to be attached to S-L or S, respectively. In another embodiment, a kit comprises at least S-L and a purification moiety M. In yet another embodiment, a kit according to the present invention comprises at least a moiety S-L-M. Optionally, kits according to the present invention comprise a product manual, one or more compounds or resins to carry out a purification step and/or suitable reaction or purification media and the like.

### Brief Description of the Figures

FIG. 1: Schematic representation of the purification processes of the invention. (A) Direct purification of an M-containing precursor comprising a purification element N; (B) Direct purification of an M-containing precursor comprising a reactive group on M, wherein using a resin covalently linked to a complementary reactive group is attached via said complementary reactive group to the reactive group on M; (C) 2-Step purification of an M-P-containing precursor comprising a purification element Q; (D) 2-Step purification of an M-P-containing precursor comprising multiple elements Q.

FIG. 2: HPLC chromatograms (UV detection 254 nm) of 2-(2-[¹⁸F]fluoroethyl)naphthalene ([¹⁸F]F-product) radiofluorination reaction solutions and by-product standards. A) Reaction 2 (see Example 3) reaction solution; B) Reaction 1 reaction solution; C) naphthylethanol; D) 2-vinylnaphthalene

FIG. 3: HPLC chromatograms (UV detection 220 nm) of the purification of radiofluorination Reaction 3 using the click chemistry after 15 and 30 min.

FIG. 4: HPLC chromatograms (radiometric detection) of the purification radiofluorination Reaction 3 using the click chemistry after 15 and 30 min. [¹⁸F]F-product = 2-(2-[¹⁸F]fluoroethyl)naphthalene.

### Detailed Description

The present invention relates to nucleophilic substitution reactions carried out under liquid phase, preferably homogeneous, reaction conditions, i.e., the substitution takes place in liquid reaction media. The novel liquid phase nucleophilic substitution and subsequent purification processes of the present invention are shown in a generalized manner in Scheme 4 below.

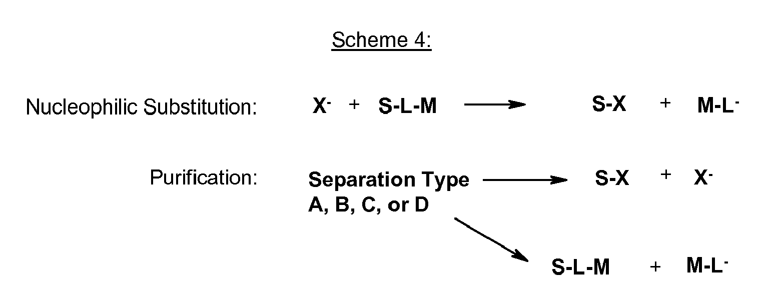

Although the preferred embodiments of the present invention refer to nucleophilic substitutions with radioactive halogen-isotopes such as ¹⁸F, any such references to radiohalogens are used by way of example only and are not intended to be limiting in any way. For example, the process can also be carried out to produce other radiopharmaceuticals, halogen-containing non-radioactive pharmaceuticals or even any nucleophilic residue-containing pharmaceuticals.

All processes of the present invention are characterized by the involvement of a special purification moiety M. In accordance with the present invention, said moiety M is covalently linked to the leaving group L of a precursor compound S-L-M that is subjected to a nucleophilic substitution reaction to attach a nucleophilic moiety X, which, e.g., may be derived prior or during the substitution reaction from a precursor X* (X* being a suitable precursor providing the nucleophile X to the reaction: A non-limiting example for, e.g., X* is a salt of X) or from a precursor X**, wherein X is a nucleophilic moiety that is transferred from X** to S during the nucleophilic reaction. In the nucleophilic substitution reactions (and the kits) of the present invention, S is a targeting substrate and L is a leaving group during said nucleophilic substitution reaction.

The purification moiety M has characteristics that allow any species that contain M to be easily separated from other even otherwise similar species that do not contain M. These characteristics are embodied in a purification element N, which is comprised within M. Various separation procedures that are effected by employing the purification moiety M are described in further detail herein below. The purification moiety M allows the efficient and convenient separation of non-reacted precursors S-L-M and the by-product M-L from the desired product S-X. It will be appreciated that the separation of M-containing species from those that do not contain M depends on the actual physical and/or chemical properties of M and can generally be performed by methods known to those skilled in the art. While not limited to these embodiments, the present invention is illustrated by describing a variety of separation types in more detail.

Separation Type A is based on liquid-liquid or solid-liquid extraction using a solution phase (liquid phase) or a resin (solid phase) that have affinity to M. In such separations, the removal of an M-containing species into a liquid extraction phase or to a solid resin generally relies on the affinity of a polar, ionic, or non-polar purification element N to the polar, ionic, or non-polar properties of the liquid extraction phase or solid resin. In general, any species that do not contain said purification moiety M (such as the desired reaction product S-X) essentially remains in the reaction mixture and is not transferred to the liquid extraction phase or solid resin, thereby achieving a separation of M-containing species from those that do not contain M. Alternatively, in embodiments of liquid-liquid extraction, M-containing species may have an affinity to the reaction mixture and essentially remain in said mixture, i.e., they are not transferred to the liquid extraction phase.

A non-limiting example of a purification element N comprised within a purification moiety M according to the present invention suitable to perform a liquid-liquid extraction is an ionic liquid moiety, which can be for example a pyridinium or imidazolium group.

The term "ionic liquid reaction phase" in the context of the present invention is to be understood as a reaction phase comprising ionic liquids. Such ionic liquids are well-known in the art (see, e.g., Thomas Welton Chem. Rev. 1999, 2071-2083). An ionic liquid is a special type of salt compound that melts at around room temperature or even below to form a liquid phase. Such ionic liquids often comprise a cationic organic moiety such as a pyridinium, imidazolium, tetraalkylammonium, or phosphonium group along with a suitable anion. Therefore, an "ionic liquid moiety" as used herein refers to a moiety that is covalently bound to, e.g., L and is, or comprises, an ion of an "ionic liquid".

In certain embodiments of the present invention, M-containing species according to the present invention are extracted into an ionic liquid phase.

As a non-limiting example, a nucleophilic substitution reaction according to the present invention is carried out in a liquid reaction phase that is relatively non-polar compared to an "ionic liquid" liquid extraction phase, and M-containing species (wherein M is or comprises N, e.g., a pyridinium or imidazolium group) are subsequently extracted into an "ionic liquid" liquid extraction phase. Species that do not contain a purification moiety M such as S-X stay in the reaction phase.

Accordingly, some embodiments of liquid-liquid extraction relate to the separation of M-containing species from species that do not contain a purification moiety M, wherein the separation relies on the affinity of said M to a liquid extraction phase.

In particular embodiments, a moiety M that is charged or species containing said charged moiety M are extracted into an "ionic liquid" phase.

In particular embodiments of solid-liquid extraction, the extraction of M-containing species relies on the affinity of said M-containing species comprising a charged moiety M to an "ionic liquid" moiety attached to a resin.

In some embodiments, the extraction of M-containing species relies on the affinity of said M-containing species comprising an "ionic liquid" moiety M to an appropriately charged different "ionic liquid" moiety attached to a resin.

In other embodiments of the liquid-liquid extraction described above, the separation of M-containing species and species that do not contain a purification moiety M is based on the affinity of M-containing species (wherein M comprises N, and N is, e.g., a pyridinium or imidazolium group) to the liquid reaction phase whereas species that do not contain a purification moiety M are extracted into a liquid extraction phase, i.e. in a particular embodiment of liquid-liquid extraction separation methods, M has an affinity for the reaction solution liquid phase rather than for the extracting liquid phase, and therefore the reaction product S-X can be extracted from the reaction solution to effect the purification.

In other words, in certain embodiments of the present invention related to the separation of M-containing species from species that do not contain a purification moiety M, the separation relies on the affinity of said M to the reaction phase.

As a non-limiting example, a nucleophilic substitution reaction according to the present invention can be carried out in an "ionic liquid" liquid medium and a subsequent liquid-liquid extraction is carried out with a non-polar solvent to separate out S-X. M-containing species (wherein M is, e.g., a pyridinium or imidazolium group) stay in the ionic liquid reaction phase.

In embodiments of solid-liquid extraction, the extraction of M-containing species relies on the affinity of said species to a solid resin (or to a group that is attached to a resin).

In particular embodiments of solid-liquid extraction, species containing a charged moiety M may be retained by an ion exchange resin. For example, a cationic (or zwitterionic) moiety M may be retained by a cation exchange resin, and similarly an anionic (or zwitterionic) moiety M may be retained by an anion exchange resin. In these particular embodiments, it is desirable for the product S-X to possess no charge or a charge opposite to that of M

Furthermore, M-containing species can also be separated from the product S-X, i.e. they can be removed from the reaction mixture, by precipitation and subsequent filtration or centrifugation because M makes them prone to precipitate under certain conditions (Separation Type B). For example, N can be PEG chains, which are prone to precipitate when added to non-polar organic solvents. Such compounds can then be easily removed by filtration or centrifugation.

M-containing species may also be separated from the product S-X by size exclusion (Separation Type C). Ultrafiltration (also known as molecular weight (MW) cut-off filtration) or size exclusion chromatography (sometimes also referred to as gel chromatography) can for example be used to remove soluble, unwanted species S-L-M or M-L, i.e., M-containing species, from a mixture comprising S-X, since M makes the non-wanted species considerably larger than S-X. For example, the PEGylated species described above can also be isolated with molecular weight cut-off (spin) filters (commonly known as ultrafiltration) or a gel filtration, e.g., with a Sephadex^{©} cartridge.

In other embodiments, M-containing species can be separated from the product S-X by a chemical reaction of a reactive group on M with a complementary resin-bound reactive group to covalently attach M to the resin, and subsequent filtration or centrifugation of the resin (Separation Type D). For example, the reactive element N on M can be an azide group and the complementary reactive group bound to a resin can be an alkyne group (or vice versa), and the groups can form a covalent attachment through the well-known Cu(I) catalyzed Huisgen 1,3-dipolar cycloaddition ("click") reaction. Similarly, the reactive group on M may also be covalently linked to a complementary reactive group of another purification moiety P, which would leave the M-P-containing species amenable for a different type of separation.

As explained briefly hereinabove, the process for preparing a pharmaceutical S-X according to the present invention comprises a "liquid phase" nucleophilic substitution reaction of a nucleophilic agent X with a precursor S-L-M. In preferred embodiments, said nucleophilic substitution reaction is a "homogeneous" nucleophilic substitution reaction. The resulting product S-X may then be optionally further reacted to yield the final product S-X'.

### Definitions

In the context of the present invention, the following definitions shall apply:

The terms "a" or "an" as used herein means "one", "at least one" or "one or more".

The nucleophilic substitution reaction according to the present invention is carried out in a "liquid phase". A liquid phase nucleophilic substitution reaction as defined herein refers either to a two phase liquid-liquid reaction, e.g., two non-miscible solvents, optionally in the presence of a phase transfer catalyst, or it refers to a "homogeneous reaction". The term "homogeneous" as used herein to describe a substitution reaction means that the reaction conditions are uniform (i.e. in contrast to a heterogeneous reaction, as, e.g., described for the prior art purifications involving solid supports). In other words, the homogeneous nucleophilic substitution reaction takes place in a single liquid phase and the reactants are dissolved within said phase during the reaction. The person skilled in the art will understand that some compounds may precipitate from the liquid reaction mixture after completion of the substitution reaction, but the latter is not to be confused with a heterogeneous nucleophilic reaction.

The term "S" or " targeting substrate" as used herein describes a compound that preferably possesses inherent properties that give it a biodistribution favorable for imaging a pathology, disease or condition. Prior to the nucleophilic substitution reaction, S is covalently linked to a leaving group L which is itself covalently bound to a purification moiety M, i.e., the species S-L-M is subjected to a liquid phase nucleophilic substitution reaction.

S can be any suitable targeting substrate chosen for the intended purpose and has generally a molecular weight of less than about 50000, about 30000, about 15000, about 10000, preferably less than about 5000 Da, more preferably less than about 2500 Da and most preferably less than about 1500 Da.

It is readily apparent that already for practical reasons, small targeting substrates are preferred, not the least because the chemistry is better defined and there are generally less functional groups that may interact/interfere with the nucleophile X in the liquid phase nucleophilic substitution reaction of the present invention. S is typically selected from the group consisting of a synthetic small molecule, a pharmaceutically active compound (i.e., a drug molecule), a metabolite, a signaling molecule, an hormone, a peptide, a protein, a receptor antagonist, a receptor agonist, a receptor inverse agonist, a vitamin, an essential nutrient, an amino acid, a fatty acid, a lipid, a nucleic acid, a mono-, di-, tri- or polysaccharide, a steroid, and the like. It will be understood that some of the aforementioned options will overlap in their meaning, i.e., a peptide may for example also be a pharmaceutically active compound, or a hormone may be a signaling molecule or a peptide hormone. Furthermore, it will be understood that also derivatives of the aforementioned substance classes are encompassed. For example, protecting groups may be attached to reactive groups of S that are not linked with other than -L-M of a compound S-L-M.

S (or, optionally, any metabolite of S or S-X, respectively), is preferably a moiety that specifically binds to a target site in a mammalian body. Specific binding in this context means that the compound S, or S-X for that matter, accumulates to a larger extent at this target site compared to the surrounding tissues or cells. For example, S may specifically bind to a receptor or integrin or enzyme that is preferentially expressed at a pathologic site within the mammalian body, or S may be specifically transported by a transporter that is preferentially expressed at a pathologic site within the mammalian body. In some embodiments, the receptor, integrin, enzyme, or transporter is exclusively expressed at a pathologic site within the mammalian body, i.e., to sites that are different or absent in healthy subjects, or *vice versa*. In this context, it will be understood that S preferably binds specifically to a receptor / or integrin / or enzyme / or transporter that is exclusively expressed or present at a pathologic site within the mammalian body and not expressed or present at a non-pathologic site, although the latter is - while no doubt highly desirable- rarely achieved in practice.

Examples for specific binding include, but are not limited to, specific binding to a site of infection, inflammation, cancer, metastases, platelet aggregation, angiogenesis, necrosis, ischemia, tissue hypoxia, angiogenic vessels, Alzheimer's disease plaques, atherosclerotic plaques, pancreatic islet cells, thrombi, somatostatin receptors, vasoactive intestinal peptide receptors, D receptors, sigma receptors, GRP receptors, peripheral benzodiazepine receptors, estrogen receptors, progesterone receptors, GLP-1 receptors, G-protein coupled receptors, serotonin transporters, neuroepinephrin transporters, dopamine transporters, LAT 1 transporters, amino acid transporters, sugar transporters, apoptotic cells, macrophages, neutrophils, EDB fibronectin, receptor tyrosine kinases, lyases, synthases, phosphatidylserine, PSMA, cardiac sympathetic neurons, and the like.

In preferred embodiments, S may be selected from the group consisting of a synthetic small molecule, a pharmaceutically active compound (drug), a peptide, a metabolite, a signaling molecule, a hormone, a protein, a transporter or enzyme substrate, a receptor antagonist, a receptor agonist, a receptor inverse agonist, a vitamin, an essential nutrient, an amino acid, a fatty acid, a lipid, a nucleic acid, a mono-, di-, tri-, or polysaccharide, a steroid, a hormone and the like. More specifically, S may be selected from the group consisting of glucose, galactose, fructose, mannitol, sucrose, or stachyose and derivatives thereof (e.g. N-Ac groups are attached or functional groups other than -L-M are protected), glutamine, glutamate, tyrosine, leucine, methionine, tryptophan, acetate, choline, thymidine, folate, methotrexate, Arg-Gly-Asp (RGD) peptides, chemotactic peptides, alpha melanotropin peptide, somatostatin, bombesin, human pro-insulin connecting peptides and analogues thereof, GPIIb/IIIa-binding compounds, PF4-binding compounds, avβ3, avβ6, or a4β1 integrin-binding compounds, somatostatin receptor binding compounds, GLP-1 receptor binding compounds, sigma 2 receptor binding compounds, sigma 1 receptor binding compounds, peripheral benzodiazepine receptor binding compounds, PSMA binding compounds, estrogen receptor binding compounds, androgen receptor binding compounds, serotonin transporter binding compounds, neuroepinephrine transporter binding compounds, dopamine transporter binding compounds, LAT1 transporter binding compounds and hormones such as peptide hormones, and the like.

In embodiments of the present invention, it will generally be preferred that S-X shows essentially the same biologically relevant features, e.g., being a targeting moiety that specifically binds to a target site in a mammalian body, as S. In other words, X essentially does not alter the targeting properties of the moiety S.

Furthermore, in another preferred embodiment, S-X may accumulate in a major organ in a manner that allows estimation of regional tissue perfusion in that organ. For example, S may accumulate in the heart of a potential heart attack patient according to regional perfusion levels, and allow for delineation of areas where the heart has obstructed coronary arteries. Similarly, S compounds reflecting perfusion in the brain could help identify areas of stroke.

The term "protein", as used herein, means any protein, including, but not limited to peptides, enzymes, glycoproteins, hormones, receptors, antigens, antibodies, growth factors, etc., without limitation, having at least about 20 or more amino acids (both D and/or L forms thereof). Included in the meaning of protein are those having more than about 20 amino acids, more than about 50 amino acid residues, and sometimes even more than about 100 or 200 amino acid residues.

The term "peptide" as used herein refers to any entity comprising at least one peptide bond, and can comprise either D and/or L amino acids. The meaning of the term peptide may sometimes overlap with the term protein as defined herein above. Thus, peptides according to the present invention have at least 2 to about 100 amino acids, preferably 2 to about 50 amino acids. However, most preferably, the peptides have 2 to about 20 amino acids, and in some embodiments between 2 and about 15 amino acids.

The term "small molecule" is intended to include all molecules that are less than about 1000 atomic units. In certain embodiments of the present invention, the small molecule is a peptide which can be from a natural source, or be produced synthetically. In other embodiments, the small molecule is an organic, non-peptidic/proteinaceous molecule, and is preferably produced synthetically. In particular embodiments, the small molecule is a pharmaceutically active compound (i.e., a drug), or a prodrug thereof, a metabolite of a drug, or a product of a reaction associated with a natural biological process, e.g., enzymatic function or organ function in response to a stimulus.

Non-limiting examples for a peptide hormone are: angiotensin, leptin, prolaktin, oxytocin, vasopressin, bradykinin, desmopressin, gonadoliberin, insulin, glucagon, gastrin, somatostatin, calcitonin, parathormon, ANF, ghrelin, obestatin, HCG, thyreotropin, thyreoliberin, follitropin, luteotropin, adrenocortikotropin, MSH, EPO, somatotropin, IGF, LH/FSH, TSH, ACTH and GH.

Because S is generally comprised within an S-L-M species, it will be understood that S refers to any form of S being suitable to take part in a selective nucleophilic substitution reaction to exchange the leaving group L (attached to S and further attached to M) against a nucleophilic agent X or a moiety / molecule / precursor comprising X. In other words, S may optionally possess other reactive groups in addition to L-M. In some embodiments, at least one of said other reactive groups has to be protected before the nucleophilic substitution is carried out. Additionally, S may be a precursor of the desired pharmaceutical, i.e., S has to be further modified after the nucleophilic substitution to obtain the desired product.

The term "L" or "leaving group" as used herein, refers to a moiety that is independently bound to both, a targeting substrate S and a purification moiety M. L is removed from S during a nucleophilic reaction whereas it still remains connected to M. Such leaving groups for nucleophilic reactions are generally known in the art.

In all embodiments of the present invention, the moiety M is attached to L. Notably, L as used herein should be understood to mean a leaving group on its own, i.e. the presence of M does not make L to a leaving group. In other words, the leaving group L without M attached to it (the skilled reader will of course understand that in the absence of M another substituent such as H will take the place of M as a covalent bound moiety) must be a suitable leaving group in a nucleophilic substitution reaction by itself. As a non-limiting an example, -O-SO₂-CH₂-M is an -L-M moiety according to the present invention. The fact that M is present in the moiety -O-SO₂-CH₂-M does not make -O-SO₂-CH₂- a leaving group because -O-SO₂-CH₃ is already a suitable leaving group of a nucleophilic substitution reaction.

Similarly, the separation of M-containing species such as -L-M depends on M and is not dependent on L (or S), i.e. L (and/or S) does not represent a purification moiety that allows the separation of L-containing species from species that do not contain a moiety L. As a non-limiting example, the separation of the M-containing species -O-SO₂-M and -O-SO₂-(C₁-C₁₅)alkyl-M (wherein M is, e.g., a polyethyleneglycol, PEG), depends on the presence of M (PEG) and not on the fact that L may or may not contain a (C₁-C₁₅)alkyl group to which M is attached.

The leaving group L is preferably selected from the group consisting of -OSO₂-R-, -N(R)₃⁺-, -N(alkyl)₂R⁺-, 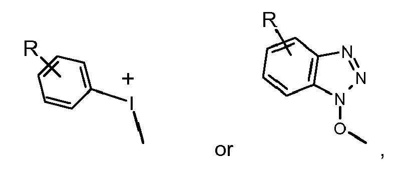 wherein R is independently chosen from C₁-C₁₅ alkyl, C₁-C₁₀ alkyl aryl, aryl, aryl alkyl, C₁-C₁₅ alkenyl, C₁-C₁₅ alkynyl, all of which may optionally be substituted, or a direct bond to M.

In the context used herein, R either is the chemical bond to M or is a moiety that is capable of forming a bond to M. Such a bond may for example be formed between a functional group that is part of R, i.e. R may be substituted with at least one functional group capable of forming a covalent bond to M such as a hydroxyl-, an aldehyde-, a keto-, a carboxyl-, an amine-, a hydrazine-, a thiol-group and the like, or alternatively R comprises a C-C double or triple bond which can be subjected to a addition reaction of M. It will be understood that any bond between L and M should be inert towards a nucleophilic substitution of X under the reaction conditions of a nucleophilic substitution reaction converting S-L-M to S-X.

In addition, if a leaving group L contains more than one R, at least one R has a direct covalent bond to M, or M is covalently bound to at least one R - either directly to a carbon atom of L or via a reactive group.

In certain embodiments, a leaving group L contains more than one R and to each R is attached at least one purification moiety M.

The term "M" or "purification moiety" as used herein refers to a moiety that is covalently bound to the leaving group "L" associated with the nucleophilic substitution reaction, i.e. the moiety L-M of a species S-L-M (and not merely L of a species S-L) is substituted by said nucleophilic reactant X. M as defined herein has characteristics that allow species that contain said purification moiety M to be separated from other species that do not contain said purification moiety M.

The purification moiety M according to the present invention comprises a functional group that can covalently connect M with the leaving group L and further comprises a purification element N (cf. Figure 1A). N is for example selected from, but not limited to the group consisting of a cationic, an anionic, a zwitterionic, an ionic liquid, a polar or a non-polar moiety; an element N that is suitable to form a chelate with metal ions, or an element N that is at least 3 times, preferably 5 times, or even 10 times or 15 times larger than S, or an element N that can lead to a precipitation of M-containing species from a reaction mixture which is adjusted to conditions so that M-containing species precipitate whereas S-X stays in solution, or an element N that comprises a reactive group capable of forming a covalent bond to a complementary resin-bound reactive group (Figure 1 B). In certain embodiments of the present invention N may comprise a carbon moiety such as optionally substituted C₁-C₁₅ alkyl, C₁-C₁₅ alkyl aryl, aryl, aryl (C₁-C₁₅) alkyl, C₁-C₁₅ alkenyl, or C₁-C₁₅ alkynyl. In other embodiments, a purification moiety M may comprise a functional group to covalently connect M with the leaving group L and a carbon skeleton (e.g. C₁- C₄ alkyl) to which at least two moieties N or in some embodiments, three or more moieties N are attached.

In a particular embodiment, the purification element N comprises a reactive group capable of forming a covalent bond to a complementary reactive group of another purification moiety P leading to a species M-P that is amenable to purification according to the methods described herein (cf. Figure 1C). In this embodiment, denoted herein as a "2-step purification", purification elements are effectively added to the M-species after the nucleophilic substitution reaction. This approach can be useful to enable efficient purification methods that might otherwise interfere with the nucleophilic substitution reaction.

A purification moiety P used for 2-step purifications according to the present invention comprises one or more than one complementary reactive groups to the reactive group of M capable of covalently connecting M and P and at least one purification element Q preferably selected from, but not limited to the group consisting of a cationic, an anionic, a zwitterionic, an ionic liquid, a polar or a non-polar moiety, a moiety that is suitable to form a chelate with metal ions, or a moiety that is at least 3 times, preferably 5 times, or even 10 times or 15 times larger than S, or a moiety that can lead to a precipitation of the M-P-containing species from a reaction mixture which is adjusted to conditions so that the M-P-containing species precipitate while S-X stays in solution, or a moiety/element Q that comprises a reactive group capable of forming a covalent bond to a complementary resin-bound reactive group. It will be understood that P typically binds only to M and not to L or S, respectively.

In a preferred embodiment of the 2-step purification, a purification moiety M comprising a reactive group is connected with a purification moiety P through a complementary reactive group of the moiety P, and the moiety P comprises more than one purification moieties Q (Figure 1 D). In certain embodiments, a purification moiety P may comprise a functional group to covalently connect P with the a reactive group of M and a carbon skeleton (e.g. C₁- C₄ alkyl) to which at least two moieties Q or in some embodiments three, four, five, six or even more moieties Q are attached.

In certain embodiments, Q may comprise a carbon moiety such as optionally substituted C₁-C₁₅ alkyl, C₁-C₁₅ alkyl aryl, aryl, aryl (C₁-C₁₅) alkyl, C₁-C₁₅ alkenyl, C₁-C₁₅ alkynyl. In some embodiments, Q comprises at least one halogen substituted carbon moieties, wherein at least one of said substituted carbon moieties are substituted by one or more, preferably 2 or more, more preferably 3 or more halogen atoms and most preferably, wherein at least one of said carbon moieties is perhalogenated. In a particular preferred embodiment, all substituted carbon moieties comprised in Q are perhalogenated. A halogen substituted carbon moiety as used in this context is preferably a C₁ to C₁₅ alkyl or C₁ to C₁₅ alkenyl. In the embodiments described above, it is preferred that the substituting halogen is fluoro. In such cases where Q is a fluoro substituted moiety, the purification process would entail a liquid-liquid extraction into a fluorous phase, or a liquid-solid extraction onto a fluorous resin.

The term "S-L-M" as used herein refers to a reactant of the nucleophilic substitution reaction. Typically, the S-L-M species according to the present invention is soluble in the liquid reaction phase under the chosen nucleophilic substitution reaction conditions. In certain embodiments, it is preferred to employ S-L-M already in its ready-to-use-form, but S-L-M may optionally be synthesized starting from S-L and M or from S and L-M prior to the nucleophilic substitution reaction using methods known to those of skill in the art. This preparatory synthesis of the precursor species may be carried out in a separate reaction mixture, or may also be carried out in the reaction mixture suitable for the nucleophilic substitution reaction, i.e. in a one-pot reaction.

The terms "X" or "reactant X" as used herein refer to any nucleophilic agent suitable to perform a nucleophilic substitution of the L-M moiety of a S-L-M precursor resulting in an S-X species. For example, X is a nucleophilic agent in its entirety or is a moiety/molecule comprising a nucleophilic group that reacts with S-L-M (e.g. an amine group). Alternatively, X may be derived from a precursor X* (e.g., a salt), or from a precursor X**, wherein X is a nucleophilic moiety that is transferred from X** to S during the nucleophilic reaction and wherein X thereby substitutes the moiety L-M of S-L-M. The reactive region of a reactant X is preferably negatively charged, but may also be a polar electron rich part of the molecule. As apparent from the foregoing, the terms "X" or "reactant X" as used herein are meant to describe all possible forms of X that may, e.g., be present in a reaction mixture to perform a nucleophilic reaction in accordance with the present invention prior to, during and after said reaction. As an example for illustrating the different possible forms of X useful in the context of the present invention, X can be an anionic form prior to said nucleophilic reaction, or can be comprised in an intermediate state during the nucleophilic reaction, and will in any case be a covalently attached group to S after the nucleophilic reaction forming the product S-X. It will be understood that the same principle extends also to the other synonyms of the species used herein, e.g., S, L, M, L-M, P, S-L-M, etc.

In particular embodiments of the present invention, X is a halogen or a halide, for example fluorine or fluoride. In other preferred embodiments, the nucleophilic agent X is or comprises a radioisotope selected from but not limited to the group of ^{99m}Tc, ¹¹¹In, ¹⁸F, ²⁰¹TI, ¹²³I, ¹²⁴I, ¹²⁵I, ¹³¹I, ³⁴Cl, ¹¹C, ³²P, ⁷²As, ⁷⁶Br, ⁸⁹Sr ¹⁵³Sm ¹⁸⁶Re, ¹⁸⁸Re, ²¹²Bi, ²¹³Bi, ⁸⁹Zr, ⁸⁶Y, ⁹⁰Y, ⁶⁷Cu, ⁶⁴Cu, ¹⁹²Ir, ¹⁶⁵Dy, ¹⁷⁷Lu, ^{117m}Sn, ²¹³Bi, ²¹²Bi, ²¹¹At, ²²⁵Ac, ²²³Ra, ¹⁶⁹Yb, ⁶⁸Ga and ⁶⁷Ga.

It will be appreciated that some of the radioisotopes listed above are not suitable to perform a nucleophilic substitution reaction on their own. Those of skill in the art will, however, know which of the listed radioisotopes may be suitable to represent the nucleophile in a nucleophilic reaction (such as ¹⁸F), and which radioisotopes have to be bound to another nucleophilic moiety suitable to substitute L-M by virtue of a nucleophilic substitution reaction.

In embodiments wherein X is a radioisotope, it is preferred if the radioisotope is a radiohalogen such as ¹⁸F, ¹²³I, ¹²⁴I, ¹²⁵I, ¹³¹I, ³⁴Cl and ²¹¹At. The most preferred radioisotope in the context of the present invention is the ¹⁸F radioisotope of fluorine. It will be understood that the radiohalogens listed above may be present in a reaction mixture as nucleophilic agents (i.e. as an anionic species or as a polar moiety, etc.) or they may be comprised within a nucleophilic agent, wherein the radioisotope is not actively involved in the nucleophilic reaction but is a part of the substituting moiety X.

Typically, and if not explicitly stated otherwise, the term "precursors" as used herein refers to at least one, more than one or all reactants of the nucleophilic substitution reaction, i.e., X, X*, X** and S-L-M.

The term "S-X" as used herein relates to the product of a liquid phase nucleophilic reaction of a precursor/reactant S-L-M with a nucleophilic agent X. The term "S-X" may encompass neutral, non-polar, polar, negatively or positively charged species. The product "S-X" may comprise protected reactive groups and/or may be subjected to further modifications that do not interact with the S-X bond such as, e.g., deprotection or modifying a different reactive group to prepare a final product S-X'.

In one preferred embodiment, S-X is a halogenated product. In yet another preferred embodiment, S-X is a radiolabelled product, preferably a radiohalogen labeled product and most preferred an ¹⁸F labeled product.

Typically, M-containing species such as S-L-M and L-M (the displaced leaving group L bound to M) contain one purification moiety M bound to L. However, it is not excluded that such an M-containing species can also contain two or even three or more purification moieties M bound to the same L. Further, it is also contemplated that a purification moiety M can contain two or even three or more purification elements N.

The term "reaction medium" as used herein typically comprises all compounds such as buffers, salts, solvents, and soluble supports to perform a nucleophilic reaction according to the present invention. It is to be understood that, optionally, the precursors S-L-M and X may additionally be present in a reaction medium prior to the nucleophilic substitution.

The term "reaction mixture" as used herein refers typically to a liquid composition which is subjected to a liquid phase nucleophilic substitution reaction according to the present invention and comprises, or is suspected of comprising, a main product and optionally by-products and non-reacted reactants. A reaction mixture may comprise additives, which are added after said substitution reaction and prior to a subsequent purification step, to create conditions more suitable to perform said purification step, e.g., slightly changing the pH by adding an acid or a base to obtain an optimized pH value for, e.g., a chelating solid-liquid extraction.

In the context of the present application, the terms "to purify", "purification", "to separate" and "separation" are used interchangeably and are intended to mean any partitioning of a mixture of two or more species based on the presence or absence of a purification moiety M, wherein at least one species that does not contain a purification moiety M remains in or is extracted into a liquid fraction and the M-containing species end up in a separate liquid or a solid fraction. Separation therefore includes, but is not limited to, a specific and selective enrichment or depletion, concentration and/or isolation of M-containing species, or *vice versa*, of a species that does not contain a purification moiety M. However, it will be appreciated that purifying is typically understood to mean a depletion of M-containing species within a liquid phase which also containing a species that does not contain a purification moiety M (, regardless of whether said non-M species that does not contain a moiety M is further modified or separated from other compounds). It is readily apparent that there may be impurities of M-containing species left in a liquid phase after the purification.

Therefore, it is to be understood that "to purify" as used herein relates to a depletion of M-containing species in a liquid phase containing at least one species that does not contain a purification moiety M of at least 40%, of at least 50%, of at least 60%, of at least 70%, of at least 80%, or of at least 90% after a purification step, although the term preferably means an even more complete depletion of the M-containing species. Thus, whenever the application refers to the terms "to purify", "purification", "to separate" or "separation", they are intended to relate to a depletion of M-containing species from a liquid phase containing a species that does not contain a purification moiety M after a purification step of 50%, 60%, 70%, 80%, 90%, preferably 95%, more preferably 99% and most preferably 100%. In cases where the purification level is not at least 95%, preferably 97%, more preferably 99% and most preferably 100% depletion of M-containing species, serial (repeat) purification procedures may be carried out on with the reaction mixture to increase the overall purification to the desired level.

The terms "soluble supported" or "soluble support" as used herein refer to a method of synthesis on soluble polymers such as polyethylene glycol. In contrast to "classical" or "solution" synthesis which refer to homogeneous reaction schemes that do not employ polymer supports, the term "soluble supported" reactions as used herein are reserved for methodologies incorporating a soluble macromolecular carrier to facilitate, e.g., product isolation.

"Soluble supports" according to the present invention are soluble macromolecular carriers. Typically, soluble supports suitable for methods of the present invention demonstrate good chemical stability and provide appropriate functional groups for easy attachment of organic moieties and exhibit solubilizing power in order to dissolve molecular entities with low solubilities and permit a general synthetic methodology independent of the physicochemical properties of target compounds. It is to be understood that soluble supports may typically exhibit not one discrete molecular weight but instead may consist of macromolecules with variable sizes/molecular weights. Suitable solid supports can be selected from but are not limited to the group consisting of polystyrene, polyvinyl alcohol, polyethylene imine, polyacrylic acid, polymethylene oxide, polyethylene glycol, polypropylene oxide, cellulose, polyacrylamide and the like.

The term "resin" as used herein refers to a solid phase, i.e. it is insoluble in the liquid used for carrying out the nucleophilic substitution reaction or during subsequent purification. Typically, a resin is a polymer, which may optionally comprise reactive groups that are attached to the surface of the resin or that are attached to the surface of the resin by a linker.

As will be appreciated from the foregoing, the present invention is *inter alia* directed to methods for preparing pharmaceuticals comprising a liquid phase nucleophilic substitution reaction, and possible downstream methods to purify the desired product from unreacted reactants.

### Methods

One embodiment is a process for preparing a pharmaceutical S-X, wherein the moiety L-M of a precursor species S-L-M is replaced by a reactant X through a liquid phase nucleophilic substitution to form said pharmaceutical S-X and a species L-M, wherein
S is a targeting substrate;
L is a leaving group covalently attached to M and further covalently attached to S prior to said nucleophilic substitution reaction; and
M is a purification moiety that is covalently bound to L and has characteristics that allow species that contain said purification moiety M to be separated from other species that do not contain said purification moiety M; and
optionally, wherein S-X is further reacted to yield the final product S-X'.

Another aspect of the present invention relates to a process for preparing and purifying a pharmaceutical S-X, wherein the moiety L-M of a precursor species S-L-M is replaced by a reactant X through a liquid phase nucleophilic substitution to form said pharmaceutical S-X and a species L-M, wherein
S is a targeting substrate;
L is a leaving group covalently attached to M and further covalently attached to S prior to said nucleophilic substitution reaction; and
M is a purification moiety that is covalently bound to L and has characteristics that allow species that contain said purification moiety M to be separated from other species that do not contain said purification moiety M; and
optionally, wherein S-X is further reacted to yield the final product S-X'; and
wherein any species which still contain said purification moiety M (M-containing species) are selectively separated from species not containing said purification moiety M, preferably S-X, by using a purification procedure.

Yet another aspect of the present invention relates to a process for purifying a pharmaceutical S-X from a liquid phase reaction mixture comprising S-X, S-L-M, and optionally L-M, wherein;
S is a targeting substrate;
L is a leaving group covalently attached to M and further covalently attached to S prior to attaching X to S by a liquid phase nucleophilic substitution reaction; and
M is a purification moiety that is covalently bound to L and has characteristics that allow species that contain said purification moiety M to be separated from other species that do not contain said purification moiety M;
by selectively separating any species which contain said purification moiety M from S-X using a purification procedure.

In particular embodiments of the present invention, the nucleophilic substitution reaction of S-L-M to S-X is carried out as a soluble supported reaction.

In preferred embodiments of the present invention, the nucleophilic reaction of S-L-M to S-X is carried out as a homogeneous nucleophilic substitution reaction.

The separation of a species that lacks a purification moiety M, such as the desired product S-X, from one or several M-containing species may be carried out using methods generally known to a person skilled in the art. Suitable examples will be described in more detail in the following section.

### Purification methods

### Liquid-liquid purification

In preferred embodiments, species that do not contain a purification moiety M can be separated from M-containing species by liquid-liquid phase extraction. Thus, M-containing species can, e.g., be removed from the reaction mixture. Alternatively, non-M-containing species (e.g. S-X) can be removed from the reaction mixture and the M-containing species essentially stay within the reaction mixture.

Those of skill in the art are generally aware of the principles of liquid-liquid extraction. Preferably, a liquid-liquid extraction according to the present invention relies on the affinity of a polar, an ionic, or a non-polar element N on the purification moiety M to a polar, an ionic, or a non-polar liquid extraction phase or reaction phase, respectively.

A purification process according to the present invention may, e.g., comprise liquid-liquid phase extraction of an M-containing species, whereas species that do not contain a purification moiety M essentially remain in the reaction mixture. It has to be understood that the term "essentially remains in the reaction mixture" as used in this context means that at least about 60%, preferably at least about 80% more preferably at least about 90% of each species that lacks a moiety M remains in the reaction mixture. Most preferably, at least about 99% or even 100% of each species containing no moiety M remain in the reaction mixture.

Accordingly, particular embodiments relate to a liquid-liquid extraction, wherein M-containing species comprising a charged moiety M are extracted into an "ionic liquid" phase.

Other preferred embodiments relate to a liquid-liquid extraction, wherein M-containing species comprising a moiety M that is an "ionic liquid moiety" are extracted into an "ionic liquid" phase.

In embodiments of the present invention wherein the nucleophilic agent X is a radioisotope, preferably a radiohalogen such as ¹⁸F, it is desirable that the extraction medium and/or the purification moiety M do not contain a non-radioactive congener of X that may undergo an exchange reaction with the radioisotope. In accordance with this principle, it will also be understood that in cases where X comprises a radioisotope, it is desirable that the extraction medium and/or the purification moiety M do not contain a non-radioactive congener of said radioisotope. The absence of an extraction medium and/or a purification moiety M that does not contain a non-radioactive congener of X in a liquid-liquid extraction avoids yielding non-radioactive analogs of the species S-X as by-products (e.g., "cold" S-X).

As a non-limiting example, if X is ¹⁸F, it is desirable that M and/or L do not contain one or more ¹⁹F atoms which may undergo an exchange reaction with the radioisotope ¹⁸F. Such fluorine exchange reactions are well-known to those skilled in the art, and can, amongst other problems, lead to lower radiochemical yields and poor specific activity of the radiopharmaceutical.

In another preferred embodiment of the present invention, the liquid-liquid phase extraction process relies on the affinity of M-containing species to an extraction phase, such as a polar or ionic liquid extraction phase, whereas species that do not contain a purification moiety M are essentially not extractable into said liquid extraction phase, i.e. the embodiments are related to a liquid-liquid extraction process, wherein species that do not contain a purification moiety M are extracted and M-containing species essentially remain in the reaction mixture. Those of skill in the art will know how to generally perform a liquid-liquid extraction. A liquid-liquid extraction may be performed one, two, and in some cases even three, four, five or more times.

In yet another preferred embodiment, the liquid-liquid extraction process relies on the affinity of M-containing species to a liquid extraction phase that is not as polar as the reaction mixture, whereas a species that does not contain a purification moiety M is essentially not extractable into said liquid phase.

A "liquid extraction phase" as used in a liquid-liquid phase extraction method described herein is to be understood as a solution to which M-containing species are extracted, i.e. transferred to, from the reaction mixture, whereas a species that does not contain a purification moiety M essentially remains in the reaction mixture, i.e. is essentially non-extractable in said liquid extraction phase. In this context, essentially non-extractable means that at least about 60%, preferably at least about 85%, more preferably at least about 90% of each species containing no moiety M remain in the reaction mixture. In some instances, it may even be possible to achieve an almost quantitative separation wherein at least about 99% or even 100% of each species containing no moiety M remains in the reaction mixture.

### Solid-liquid extraction

In another preferred embodiment of the present invention, the purification procedure comprises a solid-liquid phase extraction of M-containing species, whereas a species that does not contain a purification moiety M remains in the reaction mixture. A skilled person will generally know how to perform a solid-liquid extraction. Such an extraction may, e.g., comprise the use of beads which can be removed by centrifugation or filtration, or such an extraction may, e.g., comprise the use of columns and the like, wherein the solid phase is the stationary phase and the reaction mixture is or is present in the mobile phase. A resin can be a solid phase in accordance with the present invention. A resin can, e.g., be unmodified or can comprise one or more active and/or complementary groups attached to it. Preferably, a solid-liquid extraction according to the present invention relies on the affinity of a polar and/or an ionic M to a polar and/or ionic solid extraction phase. Alternatively, a solid-liquid extraction according to the present invention relies on a non covalent affinity between M and the solid extraction phase, wherein a combination of van der Waals, ionic and/or polar interactions is involved in the extraction process.

Furthermore, preferred embodiments of the present invention relate to processes wherein X is a radioisotope. In these cases a solid resin or a moiety attached thereon does not contain a non-radioactive congener of X that may undergo an exchange reaction with the radioisotope as explained above.

In accordance with this principle, it will also be understood when X comprises a radioisotope, it is desirable that the extraction medium and/or the purification moiety M and/or the leaving group L do not contain a non-radioactive congener of said radioisotope.

In another preferred embodiment, the solid-liquid extraction process relies on the affinity of M-containing species to an anion exchanger, a cation exchanger, or a resin to which an ion of an "ionic liquid" is attached, whereas a species that does not contain a purification moiety M essentially shows no affinity to said resin. The term "essentially no affinity" in this context means that less than 10%, preferable less than 5%, more preferably less than 1% and most preferably less than 0.05% of a species that does not contain a purification moiety M is retained on a solid resin.

In yet another embodiment, the extraction process relies on a chelating effect of an M-containing species to a metal ion bound to a resin. In such a preferred embodiment, M is for example a His-tag attached to L which can be removed by loading the reaction mixture onto a Ni-column wherein Ni²⁺ is bound to the resin of the column.

In yet another embodiment, the extraction process relies on a non-covalent affinity between M and a complementary compound bound to a resin, wherein a combination of van der Waals, ionic and/or polar interactions are involved. In such a preferred embodiment, M is for example biotin and the complementary compound bound to a resin is avidin or streptavidin.

In another preferred embodiment of the present invention, both, liquid-liquid phase extraction and solid-liquid extraction, are based on a cation-anion attraction and the purification moiety M comprises a cationic or an anionic moiety N.

### Purification by precipitation

Following the teaching of the present invention, a M-containing species can be separated from a species that does not contain a purification moiety M by means of precipitating a M-containing species, i.e., the purification procedure comprises adjusting the reaction mixture to conditions so that the M-containing species precipitate whereas a species that does not contain a purification moiety M remains soluble.

The precipitation of a M-containing species can be achieved by, e.g., adjusting the polarity, the pH, the temperature and/or the ion strength of the reaction mixture and/or by adding, e.g., specific ions to the reaction mixture so that M-containing species precipitate whereas a species that does not contain a purification moiety M remains soluble. For example, M can comprise PEG chains (representing element N), which are prone to precipitate when added to non-polar organic solvents. Alternatively, M is bound to L and comprises a chelator or complex moiety N bound to L which is precipitated upon the addition of a specific ion reducing the solubility of the M-containing species when bound to the chelator or complex. Such precipitated species can easily be removed by filtration or centrifugation.

### Purification by size exclusion

In yet another embodiment of the present invention, a purification procedure comprises separation of M-containing species from a species that does not contain a purification moiety M by size exclusion. In such a preferred embodiment, the molecular weight of the element N of the purification moiety M is about a factor of 2, 4, 6, 10, 20, more preferable 50 and most preferably 100 times greater than S. A species that does not contain a purification moiety M can therefore be separated by suitable means available in the art, such as ultra-filtration or size exclusion chromatography. An example for a purification moiety M suitable to perform such a purification procedure is a purification moiety M comprising a polyethylene glycol (PEG) moiety (element N).

### Purification by covalently binding M to a resin

A species that does not contain a purification moiety M can further be separated from an M-containing species, if the M-containing species includes an element N being a reactive group on its purification moiety M and the purification procedure comprises reacting the M-containing species through said reactive group with a resin comprising a complementary reactive group to covalently attach the M-containing species to the resin. In accordance with the present invention, the resin is a solid. The M-containing species-resin product may then be removed from a mixture comprising at least one species that does not contain a purification moiety M by well-known methods in the art, e.g., by filtration or centrifugation.

Examples for such complementary reactive groups one group of which is attached to a resin are selected from, but not limited to the group consisting of aldehyde / hydroxylamineaminooxy, azide / alkyne, azide / phosphine, activated ester / amine, alpha keto acid / hydroxylamine, biotin / streptavidin, His-tag / Ni²⁺, (primary or secondary amine) / isocyanate, amine / thiocyanate, sulfonate / thiol, sulfonate / hydroxyl, or (aldehyde or ketone)/ (amine, hydrazide, hydrazine, phenylhydrazine, semicarbazide, aminooxy or thiocarbazide).

### 2-Step purification by forming a species -M-P

In yet another embodiment of the present invention, the purification procedure can occur in 2 steps (Figure 1C-1D). In this embodiment, M comprises an a functional group that covalently connects M with the leaving group L, and element N that is a reactive group being that is capable of forming a covalent bond to a complementary reactive group of a further purification moiety P. The further purification moiety P comprises a complementary reactive group and one or more purification elements Q that can be the same as N except not being a reactive group. The purification procedure comprises reacting the M-containing species with P to form a species having a -M-P moiety, and subsequently separating any species containing -M-P from species that do not contain -M-P (such as S-X), by a purification procedure as defined above. In general, it is preferred in all embodiments of the present invention that for cases where X is or comprises a radioactive isotope, the purification moiety M does not contain a non-radioactive congener of X that may undergo an exchange reaction with the radioisotope, e.g., during the nucleophilic substitution reaction.

The skilled person knows that nucleophilic substitution fluorination reactions are often carried out well above 100 °C. At these temperatures, enhanced exchange reactions between moieties comprising non-radioactive congener of X with the radioisotope X are often observed and therefore lead to reduced specific activities of radiolabelled product and can limit the radioactive yield of the labeling reaction.

Therefore, one preferred embodiment of the present invention relates to a process wherein, if X is or comprises a radioisotope, the group M and the group L does not contain a non-radioactive congener of X that may undergo an exchange with the radioisotope, e.g., during the nucleophilic substitution reaction.

Accordingly, 2-step purification embodiments wherein M (which optionally comprises a functional group that covalently connects M with the leaving group L), and comprises N is a reactive group N that is capable of forming a covalent bond to a complementary reactive group of a moiety P after the nucleophilic substitution reaction are preferred in certain cases. In the latter case, a nucleophilic substitution reaction comprising a radioactive X or (e.g. ¹⁸F⁻ or a compound comprising a radioactive isotope) can be carried out and subsequently M-containing species are reacted under mild conditions with P (which then may comprise non-radioactive congeners of X).

### Kits

Another aspect of the present invention relates to kits for carrying out a nucleophilic substitution reaction and/or purification procedures as described herein.

It will be apparent to those skilled in the art that the precursor S-L-M as used in the methods of the present invention may be provided ready for use, or a kit may alternatively contain some reactants or elements, such as M (optionally together with S-L), or L-M (optionally together with S)and in some cases together with S only. It will be further understood that the precursors of S-L-M (M, S-L, L-M and S, respectively) will sometimes be delivered in a modified form, for example in combination with their counter ion or with a leaving group that will be replaced in the case of S-L by M, or S by M or L-M, respectively, or in the case of M or L-M a leaving group that is substituted by S-L or S. Non-limiting examples for such leaving groups are halide moieties.

According to some embodiments of the present invention, the kit may contain the compounds M, S-L, and L-M, and/or S-L-M in any suitable combination, and additionally may optionally comprise a species X or a precursor of X such as X* or X**. In particular, a radioactive species X may be supplied with the kit if it has a suitably long half life to accommodate, manufacture, release, shipping, and receipt of the kit, but it also may be omitted if radioactive X has to be produced at the site of use (e.g. by a cyclotron).

In some embodiments of the present invention, the kit may furthermore contain a purification moiety P suitable to perform a 2-step purification as disclosed herein.

Sometimes, the kit may furthermore comprise a product manual mentioning one or more suitable S-L or S moieties, respectively, or counterparts to synthesize S-L-M and reaction conditions to perform said synthesis. Optionally, the product manual may describe one or more experimental protocols how to perform a synthesis of S-L-M, and/or one or more experimental protocols how to perform a nucleophilic substitution reaction according to the present invention (i.e. one or more experimental protocols of how to perform a synthesis of S-X or S-X', respectively), and/or one or more experimental protocols to perform a purification of S-X. Moreover, the product manual may in certain embodiments further describe one or more experimental protocols how to perform a coupling reaction of M-containing species with P and/or a 2-step purifications of M-P-containing species. Furthermore, the product manual may optionally specify storage conditions for the components.

In addition, a kit for carrying out a process in accordance with embodiments of the present invention may further comprise a nucleophilic agent X or a precursor of X as described herein.

It will be understood that the compound(s) included in the kit is/are delivered as part of a single reaction mixture, or separately packaged into one or a plurality of suitable containers. It is in some instances advantageous if the kit will further comprise a liquid-soluble support and /or a suitable reaction medium.

The kits of the present invention may further comprise a liquid extraction phase or the compounds to prepare a liquid extraction phase for separating M-containing species from a species that does not contain a purification moiety M by a purification procedure as described herein.

Optionally, the kit may further comprise at least one extraction resin to separate M-containing species from a species that does not contain a purification moiety M as described herein and/or the kit may comprise compounds to achieve conditions within the reaction mixture so that the M-containing species precipitate whereas a species that does not contain a purification moiety M remains soluble. Such compounds may be acids or bases to adjust the pH, organic solvents to adjust the polarity or salts to adjust the ion strength of the reaction mixture.

In another embodiments, the kit will also include a resin comprising a complementary reactive group which is suitable to covalently bind to a reactive group on the purification moiety M of an M-containing species so as to separate M-containing species from a species that does not contain a purification moiety.

The kits of the present invention may comprise the various compounds or media as one or more solutions that are in ready to use form (i.e., all components are present in the desired concentration to carry out a method according to the present invention), or theyit may contain one or several compounds or media in the form of a concentrated solution that is to be diluted with a pre-determined amount of solvent prior to their use. The concentration of such a stock solution may be, without limiting the scope, 1.5x, 2x, 2.5x, 5x, 10x, 50x, 100x, or 1000x of the concentration of a ready to use solution. Alternatively, the kit may comprise one or several compounds or media in dry form or lyophilized form that are to be dissolved with a suitable solvent to the appropriate concentration for use in a method according to the present invention

It will be understood that all of the preferred compounds described herein, as well as the preferred media and embodiments to purify a species lacking a purification moiety M may be included in the kits of the present invention.

It is generally preferred that each component, each dried component, each stock solution or solution ready to use (such as the reaction medium or a liquid extraction phase) will be separately placed in a sealed container, although it will be apparent to those of skill in the art that other combinations and packaging options may be possible and useful in certain situations. For example, the precursor S-L-M may already be combined with the reaction mixture.

The invention is further illustrated by the following numbered embodiments.
1. A process for preparing a pharmaceutical S-X, wherein the moiety L-M of a precursor species S-L-M is replaced by a reactant X through a liquid phase nucleophilic substitution to form said pharmaceutical S-X and a species L-M, wherein
   S is a targeting substrate;
   L is a leaving group covalently attached to M and further covalently attached to S prior to said nucleophilic substitution reaction; and
   M is a purification moiety that is covalently bound to L and has characteristics that allow species that contain said purification moiety M to be separated from other species that do not contain said purification moiety M; and
   optionally, wherein S-X is further reacted to yield the final product S-X'.
2. A process for preparing and purifying a pharmaceutical S-X, wherein the moiety L-M of a precursor species S-L-M is replaced by a reactant X through a liquid phase nucleophilic substitution to form said pharmaceutical S-X and a species L-M, wherein
   S is a targeting substrate;
   L is a leaving group covalently attached to M and further covalently attached to S prior to said nucleophilic substitution reaction; and
   M is a purification moiety that is covalently bound to L and has characteristics that allow species that contain said purification moiety M to be separated from other species that do not contain said purification moiety M; and
   optionally, wherein S-X is further reacted to yield the final product S-X'; and
   wherein any species which still contain said purification moiety M (M-containing species) are selectively separated from species not containing said purification moiety M, preferably S-X, by using a purification procedure.
3. The process according to embodiment 1 or embodiment 2, wherein the liquid phase nucleophilic substitution reaction is soluble supported.
4. A process for purifying a pharmaceutical S-X from a liquid phase reaction mixture comprising S-X, S-L-M, and optionally L-M, wherein;
   S is a targeting substrate;
   L is a leaving group covalently attached to M and further covalently attached to S prior to attaching X to S by a liquid phase nucleophilic substitution reaction; and
   M is a purification moiety that is covalently bound to L and has characteristics that allow species that contain said purification moiety M to be separated from other species that do not contain said purification moiety M;
   by selectively separating any species which contain said purification moiety M from S-X using a purification procedure.
5. The process according to any one of embodiments 1 to 4, wherein the nucleophilic reaction is carried out in a homogeneous phase.
6. The process according to any one of embodiments 1 to 5, wherein the purification moiety M comprises a functional group that can covalently connect M with the leaving group L and further comprises a purification element N that is selected from a group consisting of:
   a cationic, an anionic, a zwitterionic, an ionic liquid, a polar or a non-polar moiety N;
   an element N that is suitable to form a chelate with metal ions;
   or an element N that is at least 3 times, preferably 5 times, or even 10 times or 15 times larger than S; or
   an element N that can lead to a precipitation of M-containing species from a reaction mixture which is adjusted to conditions so that M-containing species precipitate whereas S-X stays in solution; or
   an element N that comprises a reactive group capable of forming a covalent bond to a complementary resin-bound reactive group; or
   an element N that comprises a reactive group capable of forming a covalent bond to a complementary reactive group of another purification moiety P.
7. The process according to any one of embodiments 2 to 6, wherein the purification procedure comprises a liquid-liquid phase extraction where M-containing species are extracted, and a species that does not contain a purification moiety M essentially remains in the reaction mixture, or where a species that does not contain a purification moiety M is extracted, and M-containing species remain in the reaction mixture.
8. The process according to embodiment 7, wherein, if X is or comprises a radioisotope, the extraction medium does not contain a non-radioactive congener of X that may undergo exchange with the radioisotope.
9. The process according to any one of embodiments 2 to 8, wherein the extraction process relies on the affinity of M-containing species to a liquid extraction phase that is not as polar as the reaction mixture, whereas a species that does not contain a purification moiety M is essentially not extractable into said liquid extraction phase.
10. The process according to embodiment 7 or embodiment 8, wherein the extraction process relies on the affinity of M-containing species to a polar or "ionic liquid" liquid extraction phase, whereas species that do not contain a purification moiety M are essentially not extracted into said polar or "ionic liquid" liquid phase.
11. The process according to embodiment 10, wherein M-containing species comprising a charged moiety M are extracted into an "ionic liquid" phase.
12. The process according to embodiment 10 or embodiment 11, wherein M-containing species comprising an "ionic liquid moiety" are extracted into an "ionic liquid" phase
13. The process according to any one of embodiments 2 to 6, wherein the purification procedure comprises a solid-liquid phase extraction of M-containing species, whereas a species that does not contain a purification moiety M remains in the reaction mixture.
14. The process according to embodiment 13, wherein, if X is or comprises a radioisotope, a solid resin or a moiety attached thereon does not contain a non-radioactive congener of X that may undergo an exchange reaction with the radioisotope.
15. The process according to embodiment 13 or14, wherein the extraction process relies on the affinity of M-containing species to a cation exchanger or an anion exchanger, whereas a species that does not contain a purification moiety M essentially shows no affinity to said resin.
16. The process according to any one of embodiments 7 to 15, wherein the extraction process is based on a cation-anion attraction and the purification moiety M comprises a cationic moiety N.
17. The process according to any one of embodiments 7 to 15, wherein the extraction process is based on a cation-anion attraction and the purification moiety M comprises an anionic moiety N.
18. The process according to any one of embodiments 13 to 14, wherein the extraction process relies on a chelating effect of an M-containing species to a metal ion bound to a resin.
19. The process according to embodiment 18, wherein M comprises a His-tag and the metal ion attached to a resin is Ni.
20. The process according to any one of embodiments 13 to 14, wherein the extraction process relies on a non-covalent affinity between M and a complementary compound bound to a resin, wherein van der Waals, ionic and/or polar interactions are involved.
21. The process according to embodiment 20, wherein M comprises biotin and the complementary compound bound to a resin is avidin or streptavidin.
22. The process according to any one of embodiments 2 to 6, wherein the purification procedure comprises adjusting the reaction mixture to conditions so that the M-containing species precipitate whereas a species that does not contain a purification moiety M remains soluble.
23. The process according to embodiment 22, wherein the polarity, the pH, the temperature and/or the ionic strength of the reaction mixture is adjusted to conditions and/or specific ions are added so that the M-containing species precipitate whereas a species that does not contain a purification moiety M remains soluble.
24. The process according to embodiment 22 or embodiment 23, wherein the precipitated M-containing species are removed by filtration or centrifugation.
25. The process according to any one of embodiments 2 to 6, wherein the purification procedure comprises separation of M-containing species from a species that does not contain a moiety M by size exclusion.
26. The process according to embodiment 25, wherein a species that does not contain a moiety M is separated from a M species by ultrafiltration or size exclusion chromatography.
27. The process according to embodiment 25 or embodiment 26, wherein M comprises a polyethylene glycol moiety.
28. The process according to any one of embodiments 25 to 27, wherein the molecular weight of the purification moiety M is about a factor of at least 2, 4, 6, 10, 20, 50 or 100 times greater than that of S.
29. The process according to any one of embodiments 2 to 6, wherein the M-containing species comprise a reactive group on the purification moiety M and the purification procedure comprises reacting the M-containing species through said reactive group with a resin comprising a complementary reactive group to covalently attach the M-containing species to the resin, and subsequently removing the M-containing species-resin product from species that do not contain a purification moiety M.
30. The process according to embodiment 29, wherein the removal of the M-containing species-resin product is carried out by filtration or centrifugation.
31. The process according to embodiment 29 or embodiment 30, wherein the complementary reactive groups are selected from the group consisting of aldehyde / aminooxy, azide / alkyne, azide / phosphine, activated ester / amine, alpha keto acid / hydroxylamine, biotin / streptavidin, His-tag / Ni²⁺, (primary or secondary amine) / isocyanate, amine / thiocyanate, sulfonate / thiol, sulfonate / hydroxyl, (aldehyde or keto) / (amine, hydrazide, hydrazine, phenylhydrazine, semicarbazide, aminooxy or thiocarbazide).
32. The process according to any one of embodiments 2 to 6, wherein the purification occurs in 2 steps, the first being covalently attaching the moiety M through a reactive group to a second purification moiety P, wherein the moiety P comprises a complementary reactive group and at least one purification element Q.
33. The process according to embodiment 32, wherein Q comprises at least one carbon moiety that is substituted with halogen.
34. The process according to embodiment 33, wherein said halogen is fluorine.
35. The process according to embodiment 33 or embodiment 34, wherein said carbon moiety is substituted by at least one, preferably at least two, and more preferably by at least three fluorine atoms.
36. The process according to any one of embodiments 33 to 35, wherein said carbon moiety is perfluorinated.
37. The process according to any one of embodiments 1 to 36, wherein the leaving group
   L is selected from the group consisting of -OSO₂R-, -N(R)₃⁺, -N(alkyl)₂R⁺-, 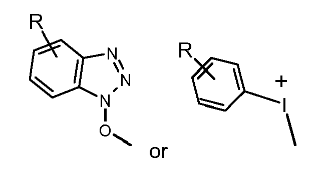 wherein R is independently chosen from optionally substituted C₁-C₁₅ alkyl, C₁-C₁₀ alkyl aryl, aryl, aryl alkyl, C₁-C₁₅ alkenyl, C₁-C₁₅ alkynyl or a direct bond to M.
38. The process according to any one of embodiments 1 to 37, wherein, if X is or comprises a radioisotope, the extraction medium does not contain a non-radioactive congener of said radioisotope.
39. The process according to any one of embodiments 1 to 38, wherein, if X is or comprises a radioisotope, the group M or the group L does not contain a non-radioactive congener of X that may undergo exchange with the radioisotope.
40. The process according to any one of embodiments 1 to 39, wherein S has a molecular weight of less than about 10.000 Da, preferably less than about 5.000 Da, more preferably less than about 2.500 Da and most preferably less than about 1.500 Da.
41. The process according to any one of embodiments 1 to 40, wherein S is a selected from the group consisting of a synthetic small molecule, a pharmaceutically active compound (drug), a metabolite, a signaling molecule, an hormone, a peptide, a protein, a transporter or enzyme substrate, a receptor antagonist, a receptor agonist, a receptor inverse agonist, a vitamin, an essential nutrient, an amino acid, a fatty acid, a lipid, a nucleic acid, a mono-, di-, tri- or polysaccharide, and a steroid.
42. The process according to any one of embodiments 1 to 41, wherein S is a targeting moiety that specifically binds to a target site in a mammalian body.
43. The process according to embodiment 42, wherein S specifically binds to a receptor or enzyme or integrin or is specifically transported by a transporter that is preferentially expressed at a pathologic site within the mammalian body, preferably wherein the receptor or enzyme or integrin or transporter is exclusively expressed at a pathologic site within the mammalian body.
44. The process according to any one of embodiments 1 to 43, wherein S binds specifically to a site of infection, inflammation, cancer, metastases, platelet aggregation, angiogenesis, necrosis, ischemia, tissue hypoxia, angiogenic vessels, Alzheimer's disease plaques, atherosclerotic plaques, pancreatic islet cells, thrombi, somatostatin receptors, vasoactive intestinal peptide receptors, D receptors, sigma receptors, GRP receptors, peripheral benzodiazepine receptors, estrogen receptors, progesterone receptors, GLP-1 receptors, G-protein coupled receptors, serotonin transporters, neuroepinephrin transporters, dopamine transporters, LAT1 transporters, amino acid transporters, sugar transporters, apoptotic cells, macrophages, neutrophils, EDB fibronectin, receptor tyrosine kinases, lyases, synthases, phosphatidylserine, PSMA, or cardiac sympathetic neurons.
45. The process according to any one of embodiments 40 to 44, wherein S is selected from the group consisting of synthetic small molecules, pharmaceutically active compounds (drugs), peptides, metabolites, signaling molecules, proteins, receptor antagonists, receptor agonists, receptor inverse agonists, vitamins, essential nutrients, amino acids, fatty acids, lipids, nucleic acids, steroids, hormones, glucose, galactose, fructose, mannitol, sucrose, stachyose, sorbose, and derivatives thereof, glutamine, glutamate, tyrosine, leucine, methionine, tryptophan, acetate, choline, thymidine, folate, methotrexate, Arg-Gly-Asp peptides, chemotactic peptides, alpha melanotropin peptide, somatostatin, bombesin, human pro-insulin connecting peptides and analogues thereof, GPIIb/IIIa-binding compounds, PF4-binding compounds, avβ3, avβ6, or a4β1 integrin-binding compounds, somatostatin receptor binding compounds, GLP-1 receptor binding compounds, sigma 2 receptor binding compounds, sigma 1 receptor binding compounds, peripheral benzodiazepine receptor binding compounds, epidermal growth factor receptor binding compounds, PSMA binding compounds, estrogen receptor binding compounds, androgen receptor binding compounds, serotonin transporter binding compounds, neuroepinephrine transporter binding compounds, dopamine transporter binding compounds, LAT1 transporter binding compounds, and any combinations thereof.
46. The process according to any one of embodiments 1 to 42, wherein S-X accumulates in a major organ in a manner that allows estimation of regional tissue perfusion in that organ.
47. The process according to any one of embodiments 1 to 46, wherein the nucleophilic reagent X is or comprises a radioisotope.
48. The process according to embodiment 47, wherein the radioisotope is selected from the group consisting of ^{99m}Tc, ¹¹¹In, ¹⁸F, ²⁰¹Tl, ¹²³I, ¹²⁴I, ¹²⁵I, ¹³¹I, ³⁴Cl, ¹¹C ³²P, ⁷²As, ⁷⁶Br, ⁸⁹Sr ¹⁵³Sm ¹⁸⁶Re, ¹⁸⁸Re, ²¹²Bi, ²¹³Bi, ⁸⁹Zr, ⁸⁶Y, ⁹⁰Y, ⁶⁷Cu, ⁶⁴Cu, ¹⁹²Ir, ¹⁶⁵Dy, ¹⁷⁷Lu, ^{117m}Sn, ²¹³Bi, ²¹²Bi, ²¹¹At, ²²⁵Ac, ²²³Ra, ¹⁶⁹Yb, ⁶⁸Ga and ⁶⁷Ga.
49. The process according to embodiment 47, wherein the radioisotope is a radiohalogen.
50. The process according to embodiment 49, wherein the radiohalogen is selected from the group consisting of ¹⁸F, ⁷⁶Br, ¹²³I, ¹²⁴I, ¹²⁵I, ¹³¹I, ³⁴Cl, ²¹¹At.
51. The process according to embodiment 50, wherein the radiohalogen is ¹⁸F.
52. The process according to any one of embodiments 1 to 46, wherein the nucleophilic reagent X is a halogen, preferably wherein the nucleophilic reagent X is fluorine or fluoride.
53. A kit for carrying out a process according to any one of embodiments 1 to 52 comprising a purification moiety M or a moiety L-M.
54. A kit for carrying out a process according to any one of embodiments 1 to 52 comprising S-L and a purification moiety M.
55. A kit for carrying out a process according to any one of embodiments 1 to 52 comprising S-L-M.
56. The kit according to any one of embodiments 53 to 55, further comprising X or a precursor of X.
57. The kit according to any one of embodiments 53 to 56, further comprising a purification moiety P suitable to perform a 2-step purification.
58. The kit according to any one of embodiments 53 to57, further comprising a product manual that describes one or more experimental protocols of how to perform a synthesis of S-X, and optionally storage conditions for the kit components.
59. The kit according to any one of embodiments 53 to 58, further comprising a liquid-soluble support.
60. The kit according to any one of embodiments 53 to 59, further comprising a liquid extraction phase to separate M-containing species from a species that does not contain a purification moiety M by a purification procedure as defined in any one of embodiments 7 to 10, 16 or 17.
61. The kit according to any one of embodiments 53 to 59, further comprising at least one extraction resin to separate M-containing species from a species that does not contain a purification moiety M by a purification procedure as defined in any one of embodiments 13 to 21.
62. The kit according to any one of embodiments 53 to 59, further comprising one or more compounds to achieve conditions within the reaction mixture as defined in any one of embodiments 22 to 24.
63. The kit according to any one of embodiments 53 to 59, further comprising at least one resin comprising a complementary reactive group which is suitable to covalently bind to a reactive group on a purification moiety M of a M-containing species as defined in any one of embodiments 29 to 31.
64. The kit according to any one of embodiments 53 to 63, further comprising a reaction medium.
65. The kit according to any one of embodiments 53 to 64, further comprising a product manual that describes one or more experimental protocols to perform a nucleophilic substitution and/or a purification procedure according to any one of embodiments 1 to 52, and optionally storage conditions for the components.
66. The kit according to any one of embodiments 53 to 65, wherein one or more components of the kit are present as solutions ready-to-use in a process according to any one of embodiments 1 to 52.
67. The kit according to any of embodiments 53 to 65, wherein one or more components of the kit are present as concentrated stock solutions that are to be diluted to the appropriate concentration for use in a method according to any of embodiments 1 to 52.
68. The kit according to embodiment 67, wherein each stock solution, independently from each other, has a concentration selected from the group consisting of 1.5x, 2x, 2.5x, 5x, 10x, 50x, 100x, and 1000x.
69. The kit according to any one of embodiments 53 to 68, wherein one or more components of the kit are present in dried or lyophilized form that are to be dissolved with solvent to the appropriate concentration for use in a process according to any one of embodiments 1 to 52.
70. The kit according to any one of embodiments 53 to 69, wherein each component, each dried component and/or each stock solution of said kit is separately placed in a sealed container.
71. Use of a purification moiety M as defined in embodiment 6 in a process according to any of embodiments 1 to 52.

It will be apparent to those of skill in the art that many modifications and variations of the embodiments described herein are possible without departing from the spirit and scope of the present invention. The present invention and its advantages are further illustrated in the following, non-limiting examples.

### Examples

### EXAMPLE 1: Synthesis of an alkyne model compound radiolabelling precursor 2-(naphthalen-2-yl)ethyl 4-(methyl(prop-2-ynyl)carbamoyl)benzenesulfonate (S-L-M)

A schematic overview of the synthesis of the radiolabelling precursor S-L-M 2-(naphthalen-2-yl)ethyl 4-(methyl(prop-2-ynyl)carbamoyl)benzenesulfonate (4) as a radiolabelling precursor is given in Scheme 5 below.

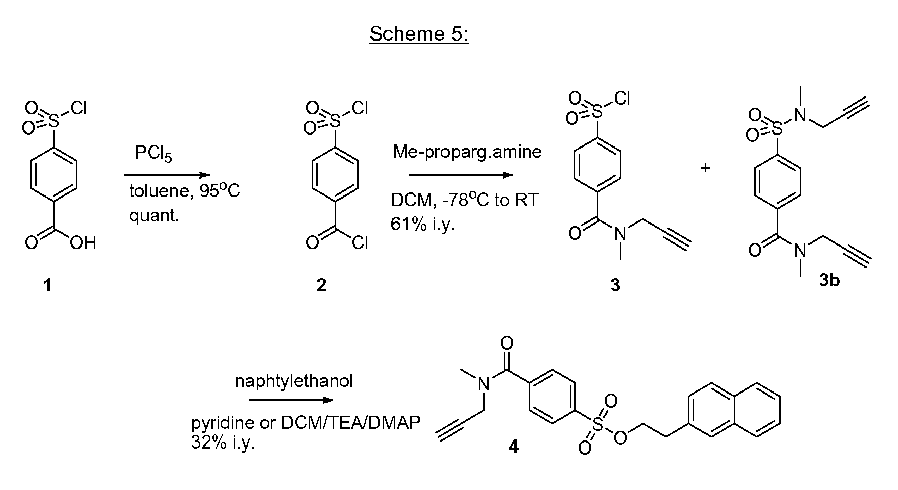

### Synthesis of 4-(methyl(prop-2-ynyl)carbamoyl)benzene-1-sulfonyl chloride (3)

4-Chlorosulfonylbenzoic acid (1) (11.94 g, 54.1 mmol) and phosphorous pentachloride (36.06 g, 173.1 mmol) were suspended in toluene and heated to 95°C external temperature for 1h. After cooling to ambient temperature the reaction mixture was poured into ice-water (250 mL), extracted with toluene (3x 150 mL), dried (sodium sulfate) and the combined organic layers evaporated in vacuo to give the intermediate 2. NMR analysis showed the reaction to be complete. The material was dissolved in a mixture of DCM (150 ml) and triethyl amine (7.53 mL, 5.48 g, 54.11 mmol). At -78°C methyl propargyl amine (4.73 mL, 3.74 g, 54.11 mmol) was dropwise added and the reaction mixture allowed to warm to room temperature over 1h. Pouring into ice-water (200 mL), extraction with DCM (3x 150 mL), drying (sodium sulfate) and evaporation of the organic solvent in vacuo afforded crude **3** (14.6 g, 53.7 mmol, 99%). After purification by automated column chromatography (SiO2, 0-2% methanol in DCM) **3** ( 8.98 g, 33.0 mmol, 61%) was isolated as a colorless solid, which was >95% purity acc. to NMR and DSC, whereas HPLC-MS indicated a purity of only >80%.

### Synthesis of 2-(naphthalen-2-yl)ethyl 4-(methyl(prop-2-ynyl)carbamoyl)benzenesulfonate (4)

3 (2.5 g, 9.2 mmol) and naphthylethanol (1.9 g, 11.0 mmol) were dissolved in DCM (5 mL). At 0°C 1 crystal of DMAP (ca. 1 mg) was added and then triethylamine (2.58 mL, 1.85 g, 18.4 mmol) was added dropwise. After stirring at ambient temperature for 24h the solvents were evaporated *in vacuo* at <30°C to give crude **4** (5.85 g, max. 9.2 mmol, quant.). Pure **4** (1.18 g, 2.9 mmol, 32%) was isolated by automated column chromatography (SiO₂, 0-10% methanol in DCM).

### Synthesis of 2-(naphthalen-2-yl)ethyl 4-(methyl(prop-2-ynyl)carbamoyl)benzenesulfonate (4)

Naphthylethanol (0.7 g, 4.0 mmol) was dissolved in pyridine (15 mL). At 0°C **3** (1 g, 3.7 mmol) was added in one batch and the mixture stirred for 1 h. Then the reaction flask was placed in a refrigerator at 5°C for 120h. The reaction mixture was poured into ice-water, stirred for 10 minutes and extracted with diethyl ether (3x 100 mL). The combined organic layers were washed with 10% aq. HCl (100 mL), water (100 mL) and dried with sodium sulfate. Evaporation of the solvent in vacuo at ambient temperature afforded crude **4** (0.44 g, 1.1 mmol, 29%).

### EXAMPLE 2. Preparation of azide-resin

### Triflyl azide

Sodium azide (20.0 g, 318.1 mmol) was dissolved in water (50 ml), DCM (80 ml) added and the mixture cooled in an ice bath. Trifluoromethylsulfonic acid anhydride (10 ml, 59.0 mmol) was added to the reaction mixture slowly over 10 min. The reaction was removed from the ice bath and stirred at ambient temperature for 2.5 h. The aqueous and organic phases were separated, and the aqueous phase was washed with DCM (3 x 30 ml). The DCM organic phases containing the triflyl azide were combined, washed with saturated Na₂CO₃ and used without further purification (160 ml).

### (a) Azide-NovaSynTG^{©} resin

NovaSynTG^{©} resin (500 mg, 0.44 mmol/g), K₂CO₃ (311 mg, 2.25 mmol) and Cu(OAc)₂ (3.0 mg, 0.015 mmol) were added to H₂O (5 ml) and MeOH (10 ml). The DCM solution of triflyl azide (8 ml) was added and the mixture closed and stored over night. The solution was filtered off, the resin was washed with DMF (6x) and DCM (6x), and then dried in vacuo over night. A Kaiser test for free amino groups conducted on the resin was negative (a few resin beads were transferred into a test tube, 3 drops each of a KCN solution in H₂O/pyridine, an 80% phenol solution in ethanol, and a 6 % ninhydrin solution in ethanol were added. The mixture was then heated for 5 min at 120°C. No blue color could be observed).

### (b) Azide-NovaPEG^{©} resin

NovaPEG^{©} resin (4.0 g, 0.6 mmol/g), K₂CO₃ (3.4 g, 24.6 mmol) and Cu(OAc)₂ (33.0 mg, 0.015 mmol) were added to H₂O (55 ml) and MeOH (110 ml). Triflyl azide in DCM (90 ml) was added and the mixture stirred over night. The solution was filtered off, the resin was washed with DMF (6x) and DCM (6x), and then dried *in vacuo* over night. A Kaiser test for free amino groups conducted on the resin (as described above) was negative.

### EXAMPLE 3. Radiofluorination; preparation of 2-(2-[¹⁸F]fluoroethyl)naphthylene (S-X)

In radiofluorination Reaction 1, [¹⁸F]Fluoride (496.2 MBq) was eluted from a QMA cartridge (equilibrated with 0.5 M K₂CO₃, washed with 10 ml H2O) with 2.0 mL of 1/3 H₂O/acetonitrile containing 5 mg Kryptofix^{©} (K222) and 1 mg K₂CO₃ into a reaction vial. The solvents were evaporated and the residue dried at 120°C under a light N₂-stream, more acetonitrile was added, and the drying process was repeated. Precursor (4) (1 mg, 2.5 µmol) in 500 µL DMSO was added to the reaction vial, the reaction stirred for 5 min at 60°C, and the finally the solution was cooled to ambient temperature. Reversed phase radio-HPLC of the product mixture indicated 64 % conversion of [¹⁸F]fluoride to 2-(2-[¹⁸F]fluoroethyl)naphthalene. HPLC conditions: C-18 reversed phase column (ACE 5 C18; 50 x 4.6 mm); gradient: 30-95% B in 10 min, 2 min 100% B, flow = 1 ml/min; solvents: A = 0.1% TFA in H2O; B = 0.1% TFA in MeCN.

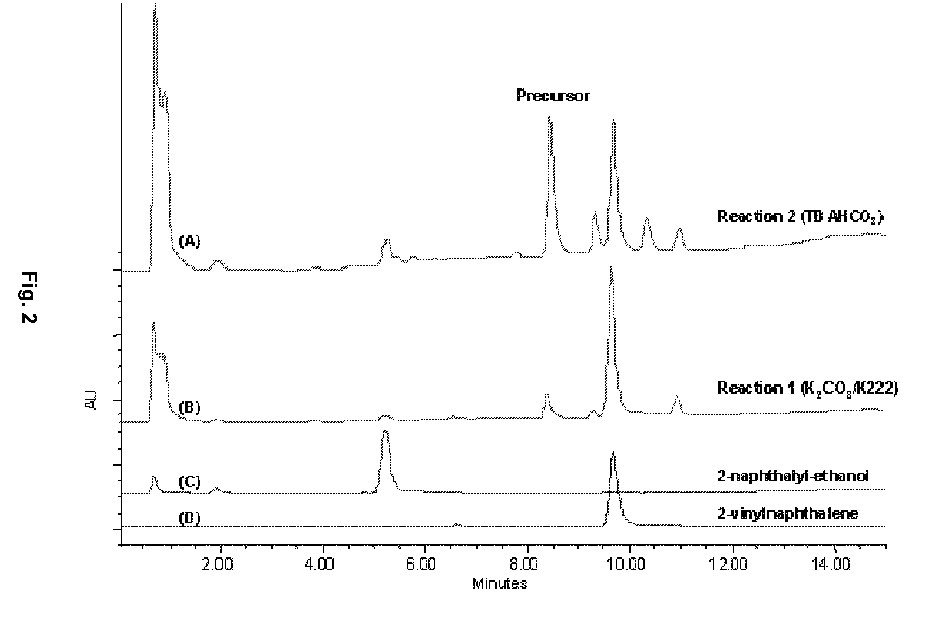

UV detection in the HPLC studies indicated degradation of the precursor to 2-naphthylethanol and 2-vinylnaphthalene, which were identified through comparison with commercially available reference compounds (Figure 2). The HPLC purity of the precursor (based on % of total HPLC peak area measured at 220 nm) in the Reaction 1 solution after radiofluorination was 25%.

In a second radiofluorination reaction (Reaction 2), the QMA cartridge was eluted with 7.5 mM TBAHCO₃ in H₂O/MeCN 1:1 (2 ml) instead of Kryptofix/K₂CO₃, and the precursor (4) (1 mg, 2.5 µmol) was added in 500 µL tBuOH/MeCN 4/1 instead of DMSO. After a similar amount of heating (60oC for 5 minutes), this reaction had lower radiochemical conversion (10%), but a higher amount of intact precursor (35% purity).

In a third radiofluorination reaction (Reaction 3), the QMA cartridge was eluted with 10.8 mM TBAOH in H₂O/MeCN 1/10 (1.1 ml) and the precursor (**4**) (1 mg, 2.5 µmol) was added in 500 µL tBuOH/MeCN 9/1. After a similar amount of heating (60°C for 5 minutes), still lower radiochemical conversion was observed (5%), but substantially more intact precursor remained (72% purity). Results for the 3 radiofluorination reactions are compiled in Table 1.

**Table 1. Radiofluorination results based on HPLC**

| Reaction | Radiofluorination conditions | % Conversion^{a} (Yield) | % Precursor after radiofluorination (HPLC purity) |
|---|---|---|---|
| 1 | K₂CO₃/Kryptofix^{©}, DMSO, 60°C, 5 min | 64 % | 25 % |
| 2 | TBAHCO₃, tBuOH/MeCN, 60°C, 5 min | 10 % | 35 % |
| 3 | TBAOH, tBuOH/MeCN, 60°C, 5 min | 5 % | 72 % |

| | | | |
|---|---|---|---|
| HPLC conditions as described in Example 3. ^{a} % Conversion = % of total HPLC peak area corresponding to the [¹⁸F] product peak; radiometric detection ^{b} % Precursor = % of total HPLC peak area corresponding to precursor; UV detection at 220 nm | | | |

### EXAMPLE 4. Purification to remove precursor via click reaction (azide resin).

In this purification step, M-containing species are linked via a complementary reactive group to a resin as prepared in Example 2.

To the radiofluorination Reaction 1 (see Example 3) was added 54.5 mg of azide-NovaSynTG^{©} resin (**a**) (see Example 2), preswelled in 400µl DMSO for 10 min, 50 µL sodium ascorbate (0.6 M) and 50 µL CuSO₄ (0.4 M). The reaction mixture was stirred at ambient temperature, and samples of the mixture were drawn at 5 and 30 min for analysis by reversed phase HPLC. Each sample was diluted 1:20 with DMSO and passed through a 0.2 µm syringe filter (PTFE membrane) before injection onto the HPLC.

A similar purification procedure was conducted on radiofluorination Reaction 2, except the azide-NovaSynTG^{©} resin (**a**) (55.2 mg) was preswelled in 400µl tBuOH/MeCN 8/2 instead of DMSO, and samples were taken at 0, 15 and 30 min for analysis by reversed phase HPLC.

A similar purification procedure was conducted on radiofluorination Reaction 3, except 49.5 mg of azide-NovaPEG^{©} (**b**) resin (see Example 2) was used in 400µl tBuOH, and samples were taken at 0, 15 and 30 min for analysis by reversed phase HPLC.

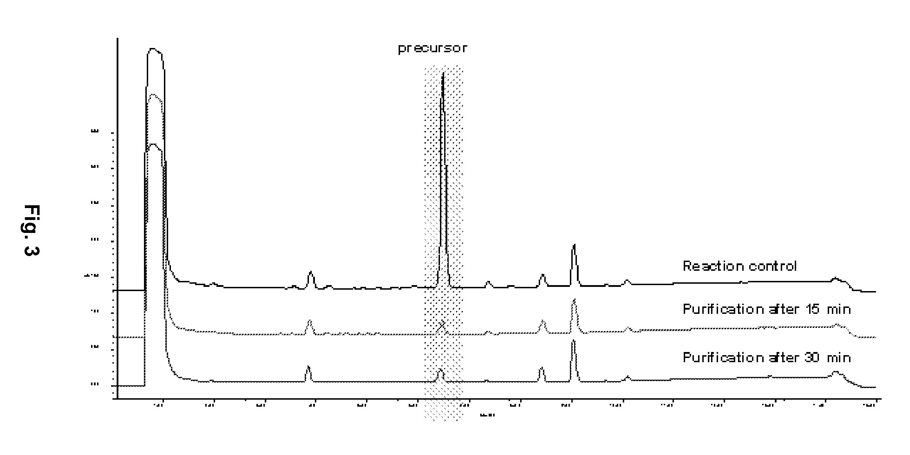

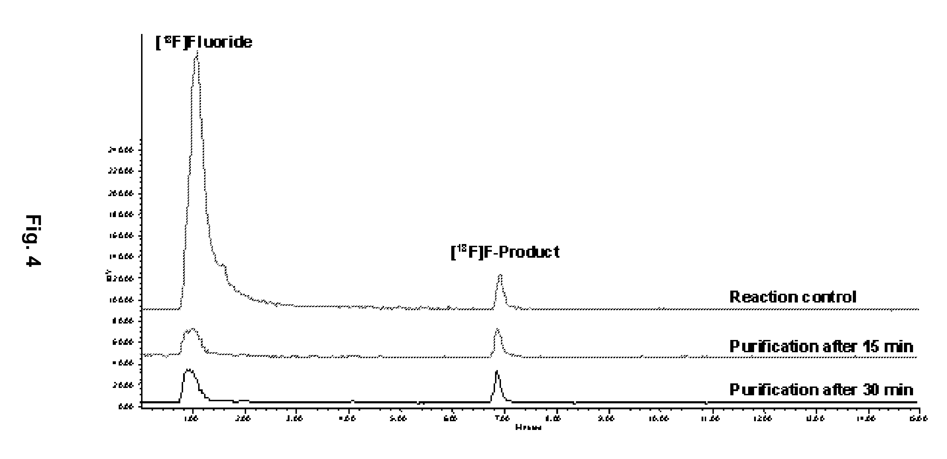

The HPLC results showed that as early as 5 minutes after adding the resin, ≥ 85% of the precursor present in the Reaction 1 solution had been depleted. Similarly, HPLC analysis of all other samples indicated >91 % removal of precursor from the reaction solutions (Table 2). Radiometric monitoring of the same HPLC injections indicated that the absolute amount of radiolabelled product was not substantially depleted by the click purification process (Table 2). Representative HPLC chromatograms for the purification studies with UV detection and radiometric detection are shown in Figures 3 and 4, respectively. A control purification experiment on a separate radiolabelling reaction equivalent to Reaction 3 using non-azide-substituted resin (i.e. resin with amino functionalities) but equivalent conditions showed 78% precursor remaining after 30 minutes (Fig. 4).

**Table 2. Radiofluorination reaction purification results. Precursor = 2-(naphthalen-2-yl)ethyl 4-(methyl(prop-2-ynyl)carbamoyl)benzenesulfonate (4). [¹⁸F]F-product = 2-(2-[¹⁸F]fluoroethyl)napthalene**

| **Sample (purification time)** | **% Precursor^{a} remaining** | **% [¹⁸F]F-product^{b} remaining** |
|---|---|---|
| Reaction 1 (5 minutes) | 14.2 | 101 |
| Reaction 1 (30 minutes) | 8.3 | 103 |
| Reaction 2 (15 minutes) | 1.6 | 126 |
| Reaction 2 (30 minutes) | 0.5 | 149 |
| Reaction 3 (15 minutes) | 5.7 | 91.5 |
| Reaction 3 (30 minutes) | 7 | 109 |

| | | |
|---|---|---|
| ^{a} % Precursor remaining = % of original precursor HPLC peak area prior to purification; UV detection 220 nm ^{b} % [¹⁸F]F-product remaining = % of original [¹⁸F]F-product HPLC peak area prior to purification; radiometric detection; decay corrected. | | |

### EXAMPLE 5. Reaction of precursor with azide resin (click reaction).

50 mg of azide-NovaPEG^{©} resin was preswelled in 500 µL tert.-amylalcohol for 10 min and 1.41 mg precursor in 400 µL tert.-amylalcohol, 50 µL sodium ascorbate (0.6 M) and 50 µL CuSO₄ (0.4 M) were added. The reaction mixture was stirred at ambient temperature, and samples were drawn at 15 and 30 min for analysis by reversed phase HPLC. Each sample was diluted 1:20 with DMSO and passed through a 0.2 µm syringe filter (PTFE membrane) before injection onto the HPLC. The HPLC chromatograms showed that only < 4 % of the original precursor amount remained after 15 minutes. There was no further decrease in precursor amount detected at 30 minutes.

## Claims

1. A process for preparing a pharmaceutical S-X, wherein the moiety L-M of a precursor species S-L-M is replaced by a reactant X through a liquid phase nucleophilic substitution to form said pharmaceutical S-X and a species L-M, wherein
S is a targeting substrate;
L is a leaving group covalently attached to M and further covalently attached to S prior to said nucleophilic substitution reaction; and
M is a purification moiety that is covalently bound to L and has characteristics that allow species that contain said purification moiety M to be separated from other species that do not contain said purification moiety M; and
optionally, wherein S-X is further reacted to yield the final product S-X'.

2. A process for preparing and purifying a pharmaceutical S-X, wherein the moiety L-M of a precursor species S-L-M is replaced by a reactant X through a liquid phase nucleophilic substitution to form said pharmaceutical S-X and a species L-M, wherein
S is a targeting substrate;
L is a leaving group covalently attached to M and further covalently attached to S prior to said nucleophilic substitution reaction; and
M is a purification moiety that is covalently bound to L and has characteristics that allow species that contain said purification moiety M to be separated from other species that do not contain said purification moiety M; and
optionally, wherein S-X is further reacted to yield the final product S-X'; and
wherein any species which still contain said purification moiety M (M-containing species) are selectively separated from species not containing said purification moiety M, preferably S-X, by using a purification procedure.

3. The process according to claim 1 or claim 2, wherein the liquid phase nucleophilic substitution reaction is soluble supported.

4. A process for purifying a pharmaceutical S-X from a liquid phase reaction mixture comprising S-X, S-L-M, and optionally L-M, wherein;
S is a targeting substrate;
L is a leaving group covalently attached to M and further covalently attached to S prior to attaching X to S by a liquid phase nucleophilic substitution reaction; and
M is a purification moiety that is covalently bound to L and has characteristics that allow species that contain said purification moiety M to be separated from other species that do not contain said purification moiety M;
by selectively separating any species which contain said purification moiety M from S-X using a purification procedure.

5. The process according to any one of claims 1 to 4, wherein the nucleophilic reaction is carried out in a homogeneous phase.

6. The process according to any one of claims 1 to 5, wherein the purification moiety M comprises a functional group that covalently connect M with the leaving group L and further comprises a purification element N that is selected from a group consisting of:
a cationic, an anionic, a zwitterionic, an ionic liquid, a polar or a non-polar moiety N;
an element N that is suitable to form a chelate with metal ions;
or an element N that is at least 3 times, preferably 5 times, or even 10 times or 15 times larger than S; or
an element N that can lead to a precipitation of M-containing species from a reaction mixture which is adjusted to conditions so that M-containing species precipitate whereas S-X stays in solution; or
an element N that comprises a reactive group capable of forming a covalent bond to a complementary resin-bound reactive group; or
an element N that comprises a reactive group capable of forming a covalent bond to a complementary reactive group of another purification moiety P.

7. The process according to any one of claims 2 to 6,
wherein the purification procedure comprises a liquid-liquid phase extraction;
wherein the purification procedure relies on the affinity of M-containing species to a liquid extraction phase that is not as polar as the reaction mixture, whereas a species that does not contain a purification moiety M is essentially not extractable into said liquid extraction phase;
wherein the purification procedure relies on the affinity of M-containing species to a polar or "ionic liquid" liquid extraction phase, whereas species that do not contain a purification moiety M are essentially not extracted into said polar or "ionic liquid" liquid phase;
wherein the purification procedure comprises a solid-liquid phase extraction of M-containing species, whereas a species that does not contain a purification moiety M remains in the reaction mixture;
comprises adjusting the reaction mixture to conditions so that the M-containing species precipitate whereas a species that does not contain a purification moiety M remains soluble;
wherein the purification procedure comprises separation of M-containing species from a species that does not contain a moiety M by size exclusion;
wherein the M-containing species comprise a reactive group on the purification moiety M and the purification procedure comprises reacting the M-containing species through said reactive group with a resin comprising a complementary reactive group to covalently attach the M-containing species to the resin, and subsequently removing the M-containing species-resin product from species that do not contain a purification moiety M; or
wherein the purification occurs in 2 steps, the first being covalently attaching the moiety M through a reactive group to a second purification moiety P, wherein the moiety P comprises a complementary reactive group and at least one purification element Q.

8. The process according to any one of claims 1 to 7, wherein the leaving group L is selected from the group consisting of -OSO₂R-, -N(R)₃⁺, -N(alkyl)₂R⁺-, 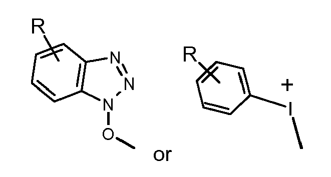 wherein R is independently chosen from optionally substituted C₁-C₁₅ alkyl, C₁-C₁₀ alkyl aryl, aryl, aryl alkyl, C₁-C₁₅ alkenyl, C₁-C₁₅ alkynyl or a direct bond to M.

9. The process according to any one of claims 1 to 8, wherein S is a selected from the group consisting of a synthetic small molecule, a pharmaceutically active compound (drug), a metabolite, a signaling molecule, an hormone, a peptide, a protein, a transporter or enzyme substrate, a receptor antagonist, a receptor agonist, a receptor inverse agonist, a vitamin, an essential nutrient, an amino acid, a fatty acid, a lipid, a nucleic acid, a mono-, di-, tri- or polysaccharide, and a steroid.

10. The process according to any one of claims 1 to 9, wherein S-X accumulates in a major organ in a manner that allows estimation of regional tissue perfusion in that organ.

11. The process according to any one of claims 1 to 10, wherein the nucleophilic reagent X is or comprises a radioisotope that is selected from the group consisting of ^{99m}Tc, ¹¹¹In, ¹⁸F, ²⁰¹Tl, ¹²³I, ¹²⁴I, ¹²⁵I, ¹³¹I, ³⁴Cl, ¹¹C ³²P, ⁷²As, ⁷⁶Br, ⁸⁹Sr ¹⁵³Sm ¹⁸⁶Re, ¹⁸⁸Re, ²¹²Bi, ²¹³Bi, ⁸⁹Zr, ⁸⁶Y, ⁹⁰Y, ⁶⁷Cu, ⁶⁴Cu, ¹⁹²Ir, ¹⁶⁵Dy, ¹⁷⁷Lu, ^{117m}Sn, ²¹³Bi, ²¹²Bi, ²¹¹At, ²²⁵Ac, ²²³Ra, ¹⁶⁹Yb, ⁶⁸Ga and ⁶⁷Ga, preferably wherein the nucleophilic reagent is ¹⁸F.

12. A kit for carrying out a process according to any one of claims 1 to 11, comprising a purification moiety M or a moiety L-M.

13. A kit for carrying out a process according to any one of claims 1 to 11, comprising S-L and a purification moiety M.

14. A kit for carrying out a process according to any one of claims 1 to 11, comprising S-L-M.

15. The kit according to any one of claims 12 to 14, further comprising a purification moiety P suitable to perform a 2-step purification.

16. The kit according to any one of claims 12 to 15, further comprising a product manual that describes one or more experimental protocols to perform a nucleophilic substitution and/or a purification procedure according to any one of claims 1 to 11, and optionally storage conditions for the components.

17. Use of a purification moiety M as defined in claim 6 in a process according to any of claims 1 to 11.
